# EUROPEAN PATENT APPLICATION

(11) **EP 4 420 594 A1**
(43) Date of publication of application: **28.08.2024**
(21) Application number: 23158510.0
(22) Date of filing: 24.02.2023
(51) Int. Cl.: A61B 3/10, G01B 9/02091

(54) **COMPACT MEMS TWO-DIMENSIONAL MEMS SCANNING MIRROR ASSEMBLY DESIGN AND RELATED ASPECTS**

(71) Applicant: Leica Microsystems Inc., Deerfield, IL 60015 (US)
(72) Inventor: HART, Robert H., Deerfield, 60015 (US)
(74) Representative: Zacco UK Ltd

(57) **Abstract**

A micro-electro-mechanical system, MEMS, two-dimensional scanning mirror assembly (310), the scanning mirror assembly having an optical design comprising a moveable MEMS scanning mirror having a reflective surface (334), a point light source (308a) for a light beam, a collimating lens assembly (516) configured to receive light from the light source and output a collimated light beam with an exit beam diameter above a threshold towards the reflective surface (334), and an objective lens assembly (510, 512) via which a collimated light beam reflected from the reflective surface exists the scanning mirror assembly. The reflective surface (334) is configured to reflect an incident collimated light beam to form a probe beam (312) which exits the mirror assembly as a telecentric beam (312) towards a telecentric image plane with a resolution better than a threshold for the telecentric beam resolution. The optics of the scanning mirror assembly are configured to provide a total track length, L, from the point light source to the telecentric image plane (700) less than 40mm.

## Description

The present disclosure relates to a compact two-dimensional MEMS-based mirror assembly and related aspects, in particular, but not exclusively, to a scanning mirror design suitable for compact optical coherence tomography, OCT, and related aspects.

Optical coherence tomography, OCT, is performed using optical apparatus to generate of a cross-sectional image of biological tissue. It is possible to achieve axial (depth) resolutions well below 8 microns using monochromatic light having a constant phase difference. Although OCT scans cannot penetrate to a great depth, they may provide depth scans of tissue in for use in medical fields such as ophthalmology and dermatology and surgical applications. It is desirable accordingly if such OCT systems can be used to generate images with depth information when probing living tissue (in vivo) in real-time as well as in other applications. To generate an image with depth information, a number of one-dimensional scans, known as A-scans, are performed along a scan line and when stacked together these A-scans create a two-dimensional image, known as a B-scan. By acquiring B-scans sufficiently closely and rapidly a volumetric image of a OCT probed sample tissue can also be obtained.

To generate three-dimensional images, OCT scanners scan a sample using a beam path across two dimensional spatial locations. Such scanning systems known in the art typically use orthogonal galvanometer mirrors to address the desired two dimensional spatial locations of the sample. To avoid Petzval curvature of the resulting interferometric image plane caused by the spatial separation of the galvanometer mirrors, relay optics are required to combine the pupil of each scanning mirror to a common pupil which can then be focused to an image plane of common optical path length for each galvanometer mirror. Alternatively, electromechanical 2D scanning mirror systems are also known in the art which use a single pupil for each scan axis, but these operate at a much reduced scan speed due to physical size restrictions of the technology, making them less desirable than systems which can scan at higher speeds.

OCT scanners are available for use in a variety of surgical procedures where depth information is advantageous. For surgical procedures on small areas, or where access to the tissue region being operated on is restricted, for example, when using OCT scanners during eye surgery, the form factor of the OCT scanner needs to be very different from the form factor of an OCT scanner where access is not restricted or no access is required to the region being probed by the OCT scanner whilst the OCT scan is ongoing.

The use of OCT scanners when performing surgical procedures creates additional design constraints for the form factor of the OCT device. On such limitation is a physical limitation on the size of the combined microscope and OCT adapter. For example, the overall height of the OCT device and any attached microscope being used by a surgeon performing a surgical procedure is limited by design so that a surgeon can view the area being scanned through the microscope whilst still accessing the area under the OCT/microscope using surgical tools for performing the surgical procedure.

A factor influencing the overall size of OCT scanners is the size of the scanning mirror assembly. MEMS, Micro-electromechanical systems, based optically reflective devices can be used to reduce the physical size restrictions of scanning mirror assembly and support higher scan rates but these have mirrors with small clear apertures. Small numerical clear aperture optical designs for OCT applications result in low lateral resolution at the image plane or require complicated optical designs due to the use of convergent light beams incident on the small aperture MEMS mirrors.

In applications such as metrology or intraoperative OCT, which benefit from a flat tomographic image plane, the spatial separation of the galvanometer mirrors require relay optics to combine the pupil of each scanning mirror to a common pupil which is then focused to an image plane. This results in large format, multi optical element scanner designs which are less desirable for intraoperative systems requiring a minimal sterile field volume. Optical designs based on small aperture reflective scanners result in low numerical aperture designs resulting in low lateral resolution or complicated optical designs due to the use of convergent light beams at high incident angles on small aperture MEMS mirrors.

The design of OCT scanning systems for surgery is accordingly subject to a variety of design constraints, particularly OCT scanning systems for eye surgery where the OCT light must access the eye interior via the pupil of the eye. Using an OCT scanner during eye surgery may impose a variety of design constraints which may be in tension with each other. In order to keep the microscope height, or other dimension of the device between the surgeon and the orientation of the tissue being operated on and depending on the form factor of the microscope, as short as possible, it is known in the art to replace the microscope objective lens with an objective lens which is part of an OCT scanner system. The OCT scanner system objective lens is aligned with the optical channel of the microscope optics and this allows the OCT scanner to use the same focal plane as the microscope uses. However, known OCT microscope adapter systems have a form factor which currently adds around 50mm or so at best to the stack height of the microscope when they are attached. It is desirable accordingly to improve the optical design of OCT adapters to better minimise the stack height (or other dimension between the surgeon and area of operation) of the microscope and OCT adapter assembly when they are fixed to each other. By reducing the stack height, access to the area being probed by the OCT scan for the surgical procedure may be improved.

### SUMMARY STATEMENTS

The disclosed technology seeks to mitigate or obviate at least some of the design limitations by providing an improved MEMS-based optical design for a scanning mirror assembly suitable for OCT scanner applications. In particular, but not exclusively, some embodiments of the disclosed technology provides an ultra-compact large numerical aperture MEMS based 2D point source optical design that incorporates a high angle of incidence at the scanning mirror to reduce the compound angle coupling when performing two dimensional, 2D, scanning. Some embodiments of the disclosed technology enable OCT images to be generated with a high lateral optical resolution from an optical path length equivalent image plane. Given the size and dimensions of the optical components of an OCT scanner adapter there is a limit to obtaining the smallest possible footprint (and volume). However, by changing at least the internal optical geometry including a design of the scanning mirror assembly, beam direction and deflection angles through the objective lens used also for the microscope, a more compact OCT scanner adapter can be achieved which sits within the footprint of a surgical microscope and which has a reduced vertical stack height. Achieving this reduction, however, is not straightforward due to limits on the dimensions of the optical components and the necessity to fit the optical geometry within such a confined space.

The following summary statements set out features, which may be preferred features in some embodiments, of the disclosed technology. The invention is defined by the accompanying claims.

The disclosed technology relates to a scanner design and related aspects which is suitable for OCT scanning but which is not limited to only OCT scanning applications. Some embodiments of the disclosed technology provide an OCT scanner having an optical configuration which supports a particularly compact housing design. Such a design is beneficial for use in surgical applications where the OCT scanner is provided as an adapter for a microscope.

According to a first aspect of the disclosed technology comprises a micro-electro-mechanical system, MEMS, two-dimensional scanning mirror assembly, the scanning mirror assembly comprising scanning mirror optics having an optical design comprising at least: a moveable reflective surface of a MEMS scanning mirror, the reflective surface being moveable in two-dimensions; a collimating lens assembly configured to receive a scanning light beam from a scanning light source and output a collimated scanning light beam towards the reflective surface, wherein the collimated scanning light beam has an exit beam diameter from the collimating lens assembly; an objective lens assembly via which the collimated scanning light beam reflected from the reflective surface exits the scanning mirror assembly, wherein the configuration of the scanning mirror assembly optics includes the reflective surface being configured to reflect an incident collimated scanning light beam towards the objective lens assembly to form a telecentric scanning beam which exits the mirror assembly telecentric towards a telecentric image plane, wherein as the reflective surface moves during a scan, the point where the telecentric scanning light beam focusses in the telecentric image plane changes; and wherein at least positions and dimensions of the scanning mirror optics within the scanning mirror assembly are configured to minimise a track length, L, from the light source to the telecentric image plane.

By configuring the scanning mirror assembly to have a track length to 40mm or less, the lateral footprint of the scanning mirror assembly is kept small, which allows the scanning mirror assembly to be housed in a scanner housing also having a small lateral footprint.

In some embodiments, the scanning mirror assembly comprises an optical block.

In some embodiments, the optical block is connected to the light source for the scanning light beam. The scanning light source may be hosted on another apparatus and the scanning light may be injected along an optical fibre from that apparatus in some embodiments.

In some embodiments, the light source for the scanning light beam in the optical assembly is a point light source, for example, the end of an optical fibre connected at one end to a light source for the scanning beam. Light from the optical fibre may enter the scanning mirror assembly via an optical fibre connector.

In some embodiments, the positions and dimensions of the scanning mirror optics within the mirror assembly define a track length L from the light source to the telecentric image plane which is less than or equal to 40mm.

In some embodiments, the scanning light is received by the scanning mirror assembly via an optical fibre end-face acting as a point light source. In some embodiments, the numerical aperture of the optical fibre acting as the point light source and a focal length of the collimating lens assembly configure the scanning light beam exit beam diameter from the collimating lens to be at least 3.1 mm.

In some embodiments, the scanning light source for example, the source of scanning light at the scanning mirror assembly, comprises an optical fibre end-face providing a point light source and a numerical aperture of the point light source and a focal length of the collimating lens assembly configure the scanning light beam exit beam diameter from the collimating lens to be at least 3.1 mm.

In some embodiments, design threshold for the telecentric beam resolution at the telecentric image plane is better than 6 microns, in other words, the image can be resolved to a degree smaller than 6 microns.

In some embodiments, the numerical aperture of the optical fibre and the focal length of the collimating lens assembly set the collimating lens exit beam design threshold to at least 3.1 mm.

In some embodiments, the combination of focal lengths of a scanning mirror objective lens and a scanning mirror field lens via which the scanning or probe beam exits the mirror assembly determines the total track length, L.

In some embodiments, the mirror assembly scans +/- 5 degrees, for example, the reflective surface (334) may be configured to move within +/- 5 degrees about each of two orthogonal axes, x, y, which intersect at an optical centre of the reflective surface of the scanning mirror in some embodiments

In some embodiments, the optical fibre has a numerical aperture of 0.14.

In some embodiments, the objective lens assembly optics comprise an objective lens and a field lens and wherein the total track length, L, is dependent on a combined focal length of the objective lens assembly optics.

In some embodiments, the objective lens comprises a F2.7 Biconvex doublet lens and the field lens comprises a F19 positive/negative meniscus doublet field lens.

In some embodiments, the optical path difference, OPD, of the telecentric scanning or probe beam output by the scanning mirror assembly has a radius of curvature is greater than 100mm.

In some embodiments, the telecentric beam is telecentric to better than an incident angle of 0.03 degrees at the telecentric image plane.

In some embodiments of the MEMS scanning mirror assembly, the reflective surface of the MEMS scanning mirror comprises a large-aperture gold-coated silicon mirror bonded to an underlying mechanical structure.

In some embodiments, the reflective surface comprises a large-aperture gold-coated silicon mirror bonded to an underlying mirror moving mechanical structure of the MEMS scanning mirror assembly.

In some embodiments, the mirror assembly is a scanning mirror in an OCT scanner apparatus and the optical fibre provides a point light source for an OCT light beam.

In some embodiments, the scanning mirror assembly is provided in the housing of an OCT scanner adapter.

In some embodiments, the OCT scanner apparatus is an OCT adapter scanner for a microscope, preferably a surgical microscope.

In some embodiments, the MEMS scanning mirror assembly is provided as an optical block in an optical coherence tomography, OCT, scanner where the scanning light beam comprises an OCT probe beam and where the light source comprises an optical fibre having an end-face which acts as a point light source for the OCT probe beam.

In some embodiments, by using an optical design which results in the internal track length within the scanning mirror assembly being 40mm or less, the housing of the OCT scanner adapter can be attached to an undercarriage of a surgical microscope and fit within at least the lateral footprint of the surgical microscope the OCT scanner housing is attached to.

In some embodiments, the OCT probe beam provides a depth scan of a sample and OCT light returned from the sample has a lateral optical resolution equal or higher than 6µm.

In some embodiments, OCT light returned from the sample along the OCT probe arm has a lateral optical resolution equal or higher than 6µm, in other words, the resolution is better than 166 line pairs per mm.

In some embodiments, the OCT scanner is provided as an OCT scanner adapter, for example, as an accessory, for a surgical microscope.

Another aspect of the disclosed technology provides a spectral-domain optical coherence tomography scanner system comprising an optical interferometer arrangement, the scanner system comprising: a coupler connected to an illuminating arm including a light source, a scan depth reference arm, a scanning arm, and a detection arm, where an illuminating light beam from the optical source enters the coupler along the illuminating arm, where the illuminating light beam is split by the coupler into a scan depth reference light beam which follows the scan depth reference arm towards a reflective surface and a scanning light beam which follows scanning arm towards a sample being scanned, where the scanning arm includes a scanning mirror assembly according to the first aspect or any one of its preferred embodiments, configured to move the scanning light beam across a sample, such that light returned from the sample is returned along the scanning arm towards the coupler, where returned light from the scanning arm and returned light from the scan depth reference arm is directed by the coupler towards an interference detector located in detector arm which is configured to detect interference between the returned light from the scanning arm and the returned light from the scan depth reference arm, and where the interference detector is configured to output data comprising an OCT interference signal of the returned light for image processing to generate a tomograph of the scanned sample.

In some embodiments, the scanner system comprises an image processor configured to process a received OCT interference signal and to output an OCT image for display.

In some embodiments, the scanner system comprises at least one display and an image processor configured to process a received OCT interference signal and to output an OCT image to the at least one display.

In some embodiments, the MEMS 2-D scanning mirror assembly comprises a MEMS 2D OCT scanning mirror comprising at least: a moveable MEMS scanning mirror having a reflective surface, an optical fibre connector configured to receive an optical fibre which acts as a point light source for an OCT beam illuminating the reflective surface, a collimating lens assembly configured to output OCT light from the point light source with an exit beam diameter of at least 3.1mm towards the reflective surface, wherein the reflective surface is configured to reflect an incident collimated OCT light beam to form an OCT scanning or probe beam which exits the mirror assembly as a telecentric beam towards a telecentric image plane with a resolution of at most 6 microns, and wherein the optics of the scanning mirror assembly are configured to provide a total track length, L, from a) an end-face of a fibre ferrule providing the point light source inserted in the optical fibre connector to b) the telecentric image plane of less than 40mm.

In some embodiments of the scanner system, an objective lens assembly is provided in the probe arm of the scanning mirror assembly to configure the telecentric OCT beam.

Another aspect of the disclosed technology comprises an optical coherence tomography, OCT, scanner system including a micro-electro-mechanical system, MEMS, two-dimensional scanning mirror assembly according to the first aspect or any of its embodiments disclosed herein.

Advantageously, some embodiments of the disclosed technology relate to an OCT scanner system comprising an OCT scanner adapter for a surgical microscope which includes an example embodiment of a MEMS scanning mirror assembly as disclosed herein. The OCT scanner adapter configuration according to the disclosed technology is compact in the sense that the optical design of the MEMS scanning mirror allows for an optical channel formed by microscope optics and an attached OCT scanner objective lens to require an optimally short housing stack height. The optical design also enables the housing of the OCT scanner adapter to be laterally compact as the optical path OCT probe light follows within the scanning mirror assembly block is less than 40mm. Some embodiments of the design also allow the resulting OCT image to be generated with a resolution of 6 microns or less.

This is useful when eye surgery requires the surgeon to use the microscope or similar device to generate an enlarged image of the area of operation using microscope optics. By providing an enlarged view of an area of operation, a surgeon is able to better see the tissue being surgically operated and can at the same time keep the patient within their arm's reach.

Some embodiments of the OCT scanner system design disclosed herein result in a combined stack height of the microscope and attached OCT scanner adapter 206 which is far shorter than previously possible. The design better balances the design constraints and allows a surgeon to view an area being scanned by the OCT scanner via an eye piece or eye pieces of the microscope and keep the area being scanned in the focal plane of the microscope optics whilst still allowing the surgeon to physically reach the scanned area to perform a surgical operation.

Other aspects of the compact design provide additional benefits. For example, the design of the scanning mirror assembly reflects the beam used for feedback on the scanning mirror position in a different optical plane from that used by the OCT probe beam, and the optical path the reference beam used to determine the position of the scanning mirror is advantageously configured to reduce the likelihood of returned light from the feedback arm contaminating the mirror position reference beam or its optical source or contaminating the OCT probe beam.

The above aspects, accompanying claims, and/or examples disclosed herein above and later below may be suitably combined with each other as would be apparent to anyone of ordinary skill in the art.

Additional features and advantages are disclosed in the following description, claims, and drawings, and it may be readily apparent to combine such features disclosed in the context of one aspect or embodiment above with those disclosed in the description someone of ordinary skilled in the art.

### DESCRIPTION OF THE ACCOMPANYING DRAWINGS

Some embodiments of the disclosed technology will now be described by way of example only and with reference to the accompanying drawings in which:
Figure 1 shows schematically the basic principles of a spectral domain OCT system;
Figures 2A and 2B show schematically front and rear perspective views of an OCT scanner adapter 206 for a microscope according to some embodiments of the disclosed technology;
Figures 3A and 3B show schematically various views of the components of an example of an OCT scanner adapter 206 according to some embodiments of the disclosed technology;
Figure 4 shows an enlarged view of the OCT scanner adapter 206 shown in Figures 3A and 3B;
Figure 5A shows schematically an example of a MEMS scanning mirror assembly optical design according to some embodiments of the disclosed technology;
Figure 5B shows schematically more detail of the input arm 518 of Figure 5A;
Figure 6 shows schematically an example of a collimating lens assembly of the input arm of Figure 5A;
Figure 7 shows schematically an example of an objective lens assembly for a MEMS scanning mirror assembly according to some embodiments of the disclosed technology.

### DETAILED DESCRIPTION

The detailed description set forth below provides examples of embodiments of the disclosed technology which are explained in sufficient detail to enable those skilled in the art to put the disclosed technology into practice.

There are two forms of OCT scanning, time-domain OCT (TD-OCT) and spectral domain OCT (SD-OCT). SD-OCT uses spectral interrogation of the spectrum at the OCT interferometer output.

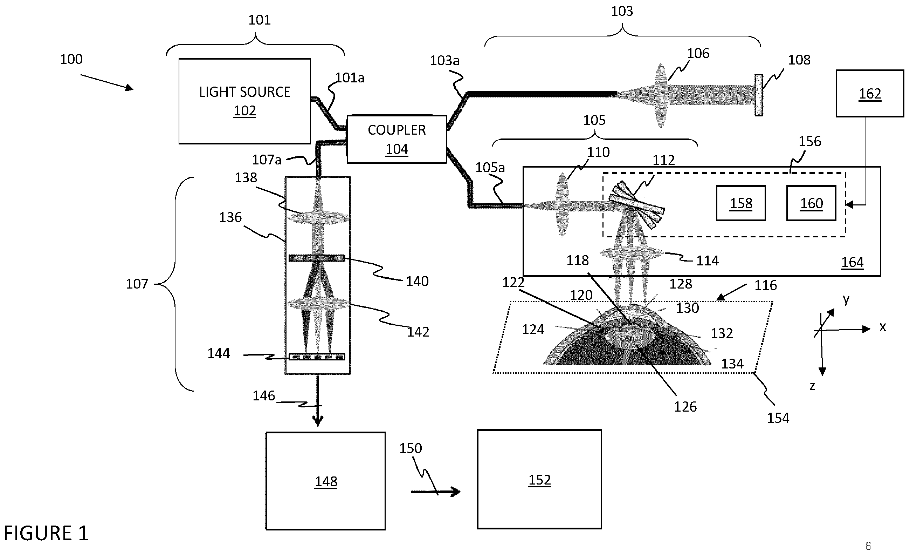

Figure 1 schematically shows the principles of operation of an example spectral domain optical coherence tomography, SD- OCT, interferometer scanner system 100 which comprises some examples of embodiments of the disclosed technology.

In the example SD-OCT system 100 illustrated in Figure 1, the SD-OCT system 100 may be used to generate optical coherence tomograms of in-vivo tissue samples 116, such as of a human eye, by using an OCT light scanning beam to probe within the tissue sample 116.

It will be apparent that the system 100 is illustrated in Figure 1 schematically and is not drawn to scale. The position of various components and their relative sizes of the SD-OCT system 100 shown in Figure 1 do not necessarily reflect their real or relative positions or sizes in example embodiments of the disclosed technology.

In the following, references to an OCT scan image or image data may refer to a one-dimensional A-scan or to a two-dimensional B-scan comprising a plurality of A-scans or to a volumetric scan image comprising a plurality of B-scans where appropriate as would be apparent to someone of ordinary skill in the art.

As illustrated in Figure 1, SD-OCT system 100 comprises a low coherence broadband optical scanning light source 102. Scanning light source 102 is suitably connected to a coupler 104 configured to split light from the light source 102 into a OCT light reference beam which follows an optical path 103a along reference arm 103 and an OCT light probe or scanning beam which follows an optical path 105a along OCT probe arm 105. OCT light returned along the reference and probe arms 103, 105 will have different phase shifts which generates interference when the returned light is recombined at the coupler 104. The combined light signal is output from the coupler along detection or output arm 107 and the light interference pattern is detected using a spectrometer 136. The output light signal 146 from spectrometer 136 is then processed by an image processor 148, for example, to apply a Fourier transform to the output light signal 146, which generates OCT scan data which can then be displayed on a suitable display 152.

The different phase shifts between the reference arm returned OCT light and the OCT returned from the probe arm which result in the interference pattern detected, arise because the OCT light is returned from one or more different structures at different depths on or within the scanned tissue sample 116. In some embodiments, the scanned tissue sample may instead comprise a different type of object of interest 112 to an in-vivo tissue sample located in an area of the human body.

The phase shift which generates the interference is affected by the different depths at which the OCT light is returned by structures within the sample scanned. The interference from the phase shift allows the signal output 146 of the spectrometer 136 to be used to generate images known as tomographs which provide a visual indication of the depth of one or more such structures in the scanned or probed area and their location in the scanned or probed area.

In some embodiments of the SD-OCT system 100 shown in Figure 1, the broadband OCT light source 102 has a central wavelength of 860nm over a bandwidth of 100nm. In some embodiments, more than one light sources 102 is used to provide the broad-band low coherence OCT scanning light over a desired bandwidth.

The probe OCT beam is returned after it has been back-scattered or reflected or otherwise returned from any structures at a certain depth in the area 116 including the tissue sample being scanned. In some embodiments, one or more or all of the optical paths 101a, 103a, 105a, 107a, are provided using suitable single-mode optical fibres and may include one or more sections where a beam following the optical fibre travels in free space.

In the embodiment of the SD-OCT system 100 illustrated schematically in Figure 1, the reference beam emerges from the coupler 104 and passes along the reference arm 103 via a collimator lens 106 before it is reflected by a translating reference mirror 108 and returned towards coupler 104 along the reference arm 103. The optical path 103a along the reference arm 103 and the optical path 105a along the probe arm 105 towards the focal plane 154 illuminating the sample or other object of interest being scanned are configured with equivalent optical path lengths. Based on the detected interference between the returned reference beam light and the returned OCT probe beam light when recombined at coupler 104, the depth(s) of any structure(s) within the sample that have back-scattered or reflected or otherwise returned the probe beam light can be determined by outputting the detected interference signal 146 to an image processor 148.

In some embodiments, the interference between the returned reference beam and returned OCT probe beam light which occurs along the output arm 107 is measured using a suitable spectrometer 136 such as that shown in Figure 1 to determine the depth of the cross-sectional image being scanned. Other embodiments of the OCT system 100 may use other techniques to measure interference and generate output signal 146.

In some embodiments of the OCT system 100, one or more or all of the optical paths 101a, 103a, 105a, 107a, comprise suitable single-mode optical fibres and/or include one or more sections where an outward or inward (relative to the coupler 104) OCT beam following an optical fibre travels in free space.

In some embodiments of the disclosed technology, although the optical path lengths followed by the reference beam and probe beam are matched, the dispersive properties of the optical fibre each beam travels along are configured to differ to improve the removal of a complex conjugate image from the OCT image output and so improve the image quality of the OCT scan image and the speed at which a complex conjugate resolved OCT scan image is obtained.

In some embodiments, the term OCT scan is used herein to refer to a B-scans and volumetric scan images of the tissue area (also referred to herein as a tissue sample) 116 which are generated using the spectral domain, SD-OCT scanner system 100.

In the example shown schematically in Figure 1 of spectral-domain OCT, the broadband light source 102 generates an OCT probe beam which illuminates an area of tissue 116 which is scanned by the OCT probe beam over a range of near-infra red wavelengths.

The spectrometer 136 shown in Figure 1 comprises a collimating lens 138 via which returned light passes through a grating 140 to generate a spectrally dependant interference pattern. The interference pattern is focussed via an objective lens 142 on line camera 144 and the image signal representing the interference pattern form the output 146 to a suitable image processing system 148. However, another suitable type of interference detector in the output arm 107 may be used in alternative embodiments.

The spectrometer 136 of the embodiment of the SD-OCT system 100 shown in Figure 1 measures spectral interference in the returned OCT light beam by measuring intensity modulations in the returned light as a function of frequency. The rate of variation of intensity over different frequencies is indicative of the location of the different reflecting layers in the samples.

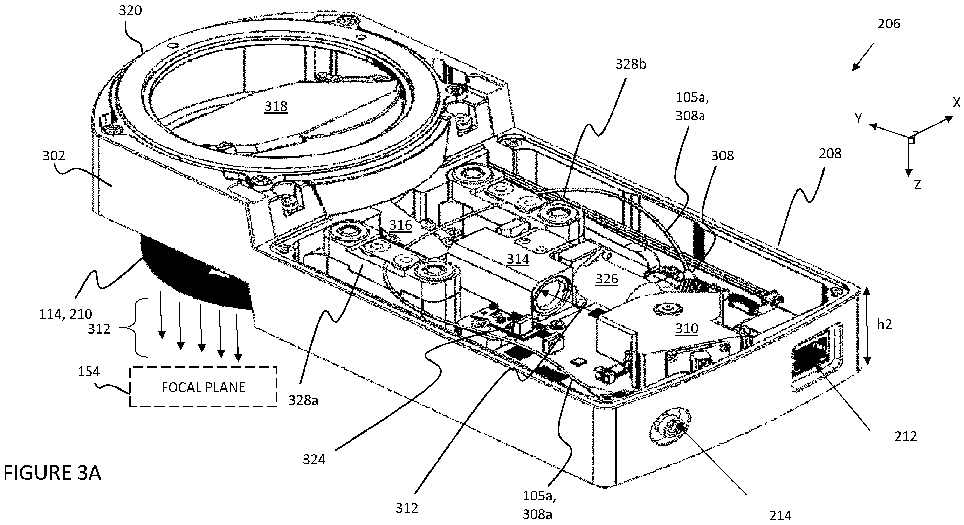

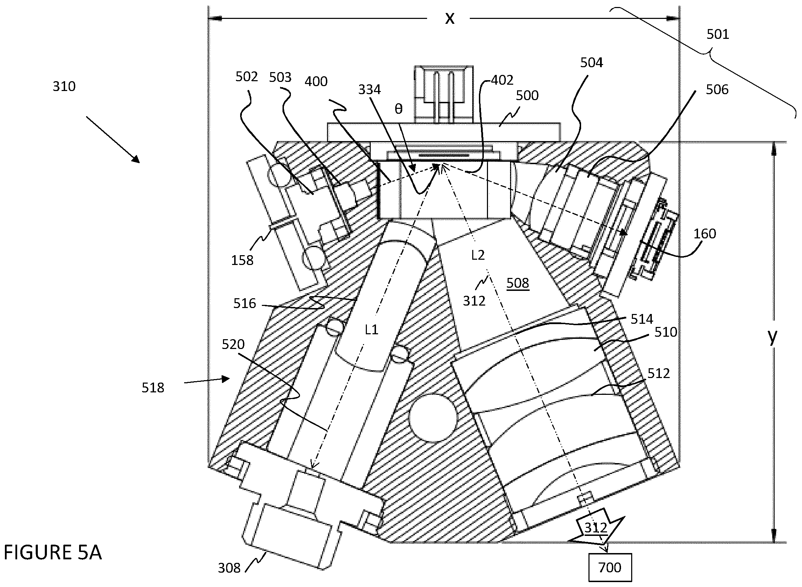

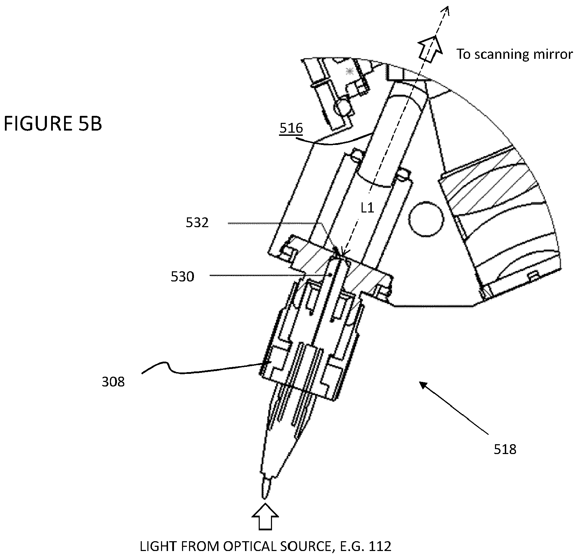

The OCT probe beam follows an optical path 105a from the coupler 104 along the OCT probe branch 105 of the coupler 104 after which it enters an OCT scanner 164. The example OCT scanner 164 shown in Figure 1 comprises a collimating lens 110, a scanning mirror assembly 310 (shown in Figures 3A and 5 for example described later below)_including a scanning mirror 112 having a reflective surface 334 (see Figure 3A or Figure 5 for example) which deflects the OCT scanning beam out of the scanner 164 via objective lens 114 towards a focal plane 154 in the scanning area 116. The scanning mirror assembly 310 includes a mirror positioning system including a secondary light source 158 which is also reflected by a scanning mirror 112 of a scanning mirror assembly towards the sample area 116 being scanned. The scanning mirror assembly includes a mirror positioning system 156 comprising a light source 158 for detecting the mirror position and a mirror position detector, PSD 160. The OCT scanner 164 also comprises an objective lens 114 which focusses the OCT scanning beam on a focal plane 154 in the area of the tissue or sample 116 being scanned.

The scanning mirror 112 may comprise a microelectromechanical system, MEMS, scanning mirror which is moved angularly by a mirror mover (not shown in Figure 1). The movement of the scanning mirror 112 moves the OCT probe beam across the sample or other object of interest being scanned and the resulting interference pattern generated is used to generate an OCT B-scan image from the system output 156..

The movement of the mirror mover is performed under the control of a controller 162. The controller 162 may be located in scanning mirror assembly which includes the scanning mirror 112 or located remotely from it.

The mirror position system 156 shown in Figure 1 comprises an optical angular displacement mirror position measurement system 156. This provides feedback on the mirror position to a controller and in some embodiments enables closed loop control of the MEMS based scanning mirror position in some embodiments.

As the scanning mirror 112 is moved in use of the OCT scanner 164 by the mirror mover mechanism under the control of the controller 162 to guide the OCT beam along a scan path. After reflection by the mirror 112, the OCT probe beam passes through a telecentric objective lens 114 which focusses the OCT probe beam at different locations in a focal plane 154 at the sample tissue 116 which is being scanned. As shown schematically in Figure 1, the focal plane 154 is illustrated as lying in a notional x-y plane, with depth information being provided orthogonally along the z-axis.

The telecentric objective lens 114 via which the probe beam passes to reach sample 116 and via which returned probe beam light also passes is shown in Figure 1 with three example emerging telecentric beams which focus on different locations within the focal plane 154, which lies in the x-y plane as shown in Figure 1. Each of the example emerging telecentric beams results from a different position of the scanning mirror assembly 112, in other words, Figure 1 is showing schematically by way of example only three sequential tele-centric beam positions. This is to show schematically how, as a B-scan or volumetric scan progresses, the telecentric OCT scanning or probe beam is moved to illuminate different areas.

The scanned area comprises a sample of tissue 116. In Figure 1 this comprises tissue of an eye 116 which may be an in vivo or in vitro tissue sample. Other types of human or animal tissue may be scanned in vivo or in vitro in other uses of the OCT scanner system where a OCT scan image may be useful to visualise internal structures at a various depths in the tissue.

For example, as shown in Figure 1, eye 116 is shown schematically and comprises a pupil 118, surrounded by an iris 120, behind which sits a posterior chamber 122 and zonal fibres 124 and in front of which are the lens of the eye 126 and cornea 128. Figure 1 also shows the anterior chamber 130 of the eye as well as the ciliary muscle 132 and suspensory ligament 134 which may all be scanned using an OCT-system such as OCT system 100 and shown as internal structures in an tomogram presented on a display 152.

The likelihood of a successful outcome from a surgical procedures performed on tissue such as the human eye, or the eye of another creature, where there is very limited access may be improved by using OCT. The OCT system 100 may be used in some embodiments to create images based on two or three-dimensional scans of region of an eye 116 as it is being operated on and these can be presented in real-time to the person performing the operation. This can allow the depth of any procedure being performed is better understood as the surgery takes place. Providing this depth information for an area subject to a surgical procedure in real-time may help a surgeon avoid making an incision which is too deep (which may damage underlying tissue unnecessarily) or one which is too shallow (in which case the operation may not be a success and/or the tissue being operated on may take longer to heal).

As is shown schematically in Figure 1, the interference signal output 146 of the spectrometer 136 of the OCT system 100 is subject to post-processing by an image processor 148. For example, the signal output 136 may be image processed using a Fourier transform or other suitable signal transform on the OCT scan. This may initially generate a warped OCT scan image which may then be subject to additional image processing to de-warp the OCT image before the OCT scan image 150 is output to a suitable display 152. Some embodiments of the OCT scanner system 100 may also use image processing to remove complex conjugate artefacts to enhance the depth range of the images obtained.

Display 152 may be part of the apparatus hosting the SD-OCT system 100 performing the image processing or a different apparatus. Some example embodiments of the disclosed technology generate a series of OCT scan images 148 using an OCT probe light beam sufficiently quickly to provide a live-stream video comprising OCT scan images 150 on display 152. Display 152 may be a near-eye display in some embodiments. In some embodiments display 152 may be a large display system comprising a plurality of displays to present information both to the surgeon and/or to others in the operating theatre. A display 152 may be integrated into the SD-OCT system 100 or be external to it.

One or more of the components shown in Figure 1 forming the OCT scanning system 100 may be housed separately from the optics forming the OCT scanner apparatus 164. By separating out the OCT scanner optics the OCT scanner 164 may have a more compact form-factor. A more compact OCT scanner 164 can be better positioned in close proximity to the sample areas being scanned.

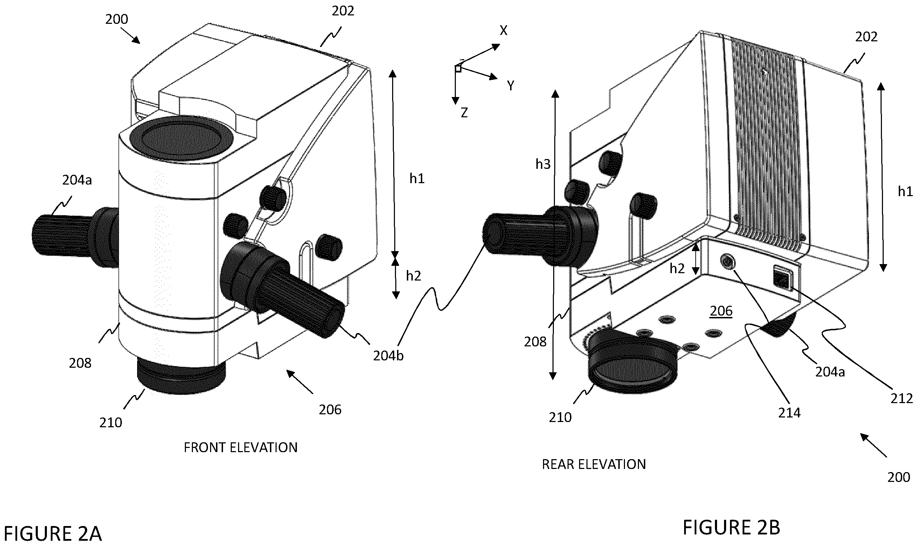

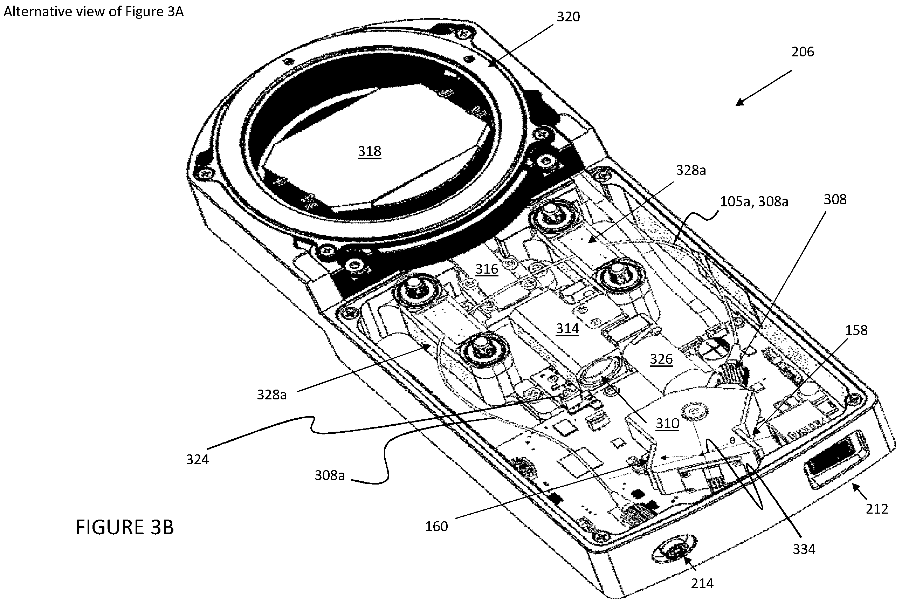

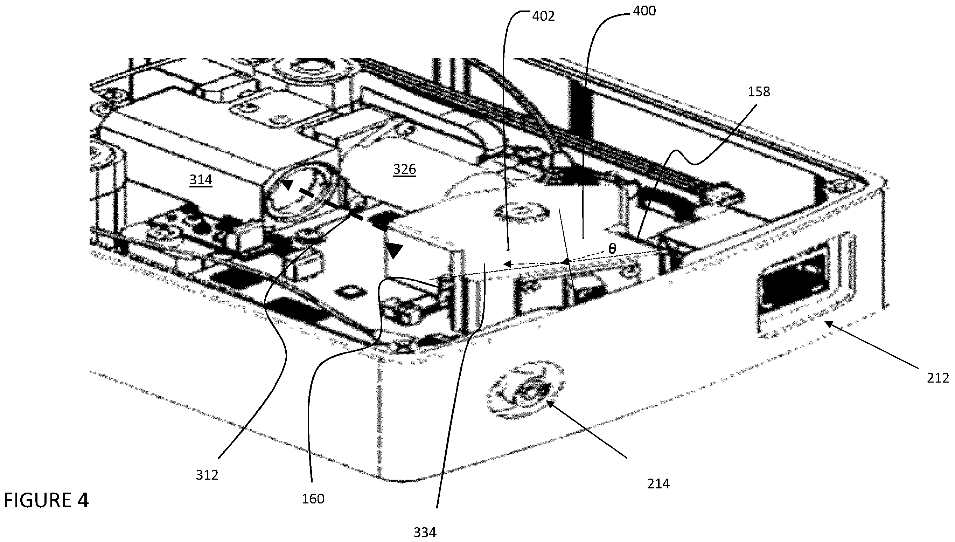

In some embodiments, the OCT scanner system 100 comprises an OCT scanner 164 provided as an adapter for a microscope, for example, the OCT scanner adapter 206 for the microscope 200 shown schematically in Figures 2A and 2B. In some embodiments, the microscope 200 comprises a surgical microscope suitable for use during surgical procedures. The housing 202 of the microscope 200 has an undercarriage configured to accept one or more microscope accessories in some embodiments, which allows an OCT scanner adapter 206 to be attached to the under carriage of the microscope housing. The OCT scanner adapter optics objective lens 114, may then also function as the microscope objective lens 210 (see also Figures 3A, 3B, and 4 of the drawings).

### EXAMPLE(S) OF MICROSCOPE SYSTEM WITH AN OCT SCANNER ADAPTER

Figures 2A and 2B show schematically front and rear perspective views of an OCT scanner adapter 206 for a microscope, in other words of an OCT scanner microscope accessory 206, according to some embodiments of the disclosed technology. The term OCT scanner adapter is herein to refers to a OCT scanner adapter microscope accessory. References to the OCT scanner adapter may also refer to an apparatus including an integrated OCT scanner adapter in some embodiments of the disclosed technology.

Figures 2A and 2B show how the OCT scanner adapter 206 of Figures 3A, 3B and 4 may be fitted to the undercarriage of microscope 200. The OCT scanner adapter 206 microscope accessory may be retrofitted to a microscope 200, for example, by removing any existing microscope accessories from the attachment points located on the undercarriage of the microscope and using their microscope attachment points to instead attach the OCT adapter 206 to the undercarriage of the microscope 200. Once suitably fixed in place, the form factor of the OCT scanner adapter aligns the objective lens 210 with at least one of the optical channels of the microscope optics, for example, a rear channel or a channel used by a microscope camera. In some embodiments, the undercarriage of the OCT adapter may also provide attachment points for accessories to be added.

In some embodiments, the OCT scanner adapter 206 has a form factor which is vertically compact so that it does not add too much height h2 to the microscope height h1 that it is attached to in use. By reducing the additional vertical height h1 of the OCT scanner adapter 206, the ease of access to the scanned area during use of the scanner when generating cross-sectional images of the scanned area 116 whilst the microscope is concurrently used is improved. The OCT scanner adapter 206 is also laterally compact. This means that when attached to the microscope 200 it does not unduly hinder surgical access to the area of tissue being scanned which allows a surgical procedure to be performed at the same time.

In the description below, reference is made to height in the context that an OCT scanner adapter 206 will be used to scan a tissue sample 116 from a location above the tissue sample, such as may result when the OCT scanner adapter 206 is mounted to the undercarriage of the surgical microscope 200.

Some embodiments of the OCT scanner 206 described herein may retain a similarly compact form factor and be used in other contexts. Moreover, the OCT scanner 206 may be provided in some embodiments integrated into another apparatus such as microscope 200. In some embodiments, OCT scanner 206 may be distributed as an optional accessory for such apparatus such that it may be distributed and sold independently of the microscope it is later attached to when in use. Accordingly, unless the context clearly prohibits it, references to height may apply equally to other dimensional directions of the OCT scanner which are substantially or approximately orthogonal to the plane of the OCT objective lens and any apparatus to which the OCT scanner is attached and the orientation of the OCT scanner and microscope stack may differ also depending one or more of a patient orientation and configuration of microscope optics and eyepiece location.

In other words, reference to height in the context of overall "height" is merely based on the assumed orientation of the OCT scanner and microscope relative to a supine patient that when surgery is being performed on the patient. Whilst a patient is supine, a surgeon may access the area being operated on below an embodiment of an OCT scanner adapter 206 according to the disclosed technology whilst at the same time having physical access to the eyepiece of the microscope 200 to which the OCT scanner 206 is attached. This geometric configuration may be varied in some embodiments depending on the configuration of the microscope optics and/or orientation of the patient and/or location of the area being operated on. Accordingly, in the description below references to the height and/or combined stack height of the microscope and OCT scanner adapter 206 may also refer to other dimensions of the microscope and OCT scanner adapter 206 which act as a constraint on the form factor of the OCT scanner where this would be apparent to someone of ordinary skill in the art.

Returning now to Figures 2A and 2B, microscope optics housed in a microscope housing 202 form an optical channel providing a view of an area below an objective lens 210 of an OCT scanner adapter 206. In some embodiments, the objective lens 210 provided by the OCT scanner adapter 206 for the microscope 200 comprises objective lens 114 of the OCT scanner system 100 shown schematically in Figure 1. References to objective lens 210 in the description may accordingly refer to the objective lens 114 of an OCT system 100 which includes a different type of OCT scanner 164 unless the context clearly limits the reference to use of the OCT scanner as an adapter or accessory for a microscope.

In the example embodiment of the OCT scanner adapter 206 shown in Figure 2A, the OCT scanning optical design has a compact form factor that adds the least possible additional height h2 to the height h1 of the microscope optics housing 202.

Also shown in Figures 2A and 2B are microscope handles 204a, b, which help position the microscope 200 above the area to be scanned (and viewed). The OCT scanner adapter 206 comprises a housing 208 which is fixed to the under carriage of the microscope 200 as shown. However, as mentioned above, in some embodiments, the OCT scanner adapter 206 may have a different configuration and/or orientation in use. Such different configurations and/or orientations of the OCT scanner adapter 206 in use may also implement the compact principles of the OCT scanner design disclosed herein.

Figure 2B shows a different, rear elevation, view of the OCT scanner adapter 206 shown in Figure 2A. The rear elevation view shows a data and/or power port 212, for example, an RSJ45 Ethernet port or USB port, and an optical port 214. Port 212 supplies power to the OCT scanner adapter 206 and in some embodiments may comprise a Power over Ethernet port.

OCT scanning light is returned from the OCT scanner adapter 206 via the optical port 214 in some embodiments to the interferometry components of a OCT scanning system 100 such as that shown in Figure 1.

For example, in the embodiment of Figure 1, the returned OCT light from sample 116 passes back via objective lens 210, 114 and is output along the optical fibre 308a via the optical port 214. The optical fibre 308a and the optical path through the mirror lens assembly and other optical components of the OCT scanner adapter 306 which lie along the optical path followed by the OCT scanning beam towards the sample being scanned accordingly form part of the probe arm 105 of the FD-OCT system 100.

After emerging and illuminating the sample tissue 116 being scanned, OCT light is reflected, back-scattered or otherwise returned. The returned OCT light then passes back through the coupler 104 where it interferes with light returned from reference arm 103. The returned OCT and reference beams then propagate along output arm 107 to spectrometer 136 which outputs the OCT and reference beam light interference signal 136 for image processing in order to generate the OCT imaging data 146 which is presented on display 152.

In some embodiments of the disclosed technology, such as, for example, that shown in Figures 1, 2A and 2B, the returned OCT light is exported from the OCT scanner adapter 206 via the optical port 214 to the coupler 104 via which it passes on to spectrometer 136 of the spectral OCT system 100.

The OCT scanner housing 208 including the objective lens 210 adds height h2 to the height h1 of the microscope 200 in the embodiment shown in Figures 2A and 2B. The additional stack height introduced by attaching the OCT scanner adapter 206to the microscope housing 202 H2 is minimised by using an optical design for the OCT scanner optical components inside the OCT scanner adapter 206 according to embodiments of the disclosed technology.

For example, some embodiments of the optical component design of an OCT scanner adapter 206 may have the optical design which is shown schematically in Figures 3A, 3B, 4 and 5A and 5B.. This optical design lifts the OCT beam emerging from the scanning mirror assembly 310 by a minimal amount up out of the plane of the objective lens 114, 210 before the OCT emerges via the objective lens 114, 120. This allows the additional stack height h2 of the OCT scanner adapter to be less than 40mm and in some embodiments the additional stack height h2 is 36mm or less.

It will be appreciated that Figures 2A and 2B are not to scale, and that the x-y-z axis shown in the Figures is schematic and illustrative only of the general front and rear perspective views. As shown in Figures 2A and 2B, the microscope housing body stack height is h1, and is aligned with the Z axis, whereas the microscope housing base and the OCT scanner adapter 206is predominantly aligned with the X-Y horizontal plane. The under-carriage mounted OCT scanner system stack height h2 is also aligned with the Z-axis. This results in the full stack height h3 of the body of microscope which houses the microscope optics combined with the under-carriage mounted OCT scanner being determined by h1 and h2. Preferably the combined height h3 of h1 + h2 = h3 is sufficiently short so that the microscope can be positioned to allow its operation by a user who is also performing surgery on or through an area including the focal plane of the microscope objective lens 210 via which the OCT beam which emerges from the microscope. The OCT optical design allows h2 to be minimised to 36 mm whilst still maintaining a suitable exit beam diameter of, for example, 10.6 mm in some embodiments and still having a stack height of 36mm or less.

Accordingly, by using an optical design for the scanning mirror assembly optics according to embodiments of the disclosed technology, the combined stack height h3 = h1 + h2 can be made much shorter than was possible with previous optical design configurations..

By reducing the stack height as much as possible, the microscope can be better positioned for surgery. For example, it may be positioned far enough from an in-focus tissue sample to allow access to the tissue sample being operated on by a user yet close enough to conform with typical human physical form factors. In other words, the OCT adapter height h2 is preferably reduced as much as possible to allow conventional operation of the microscope by the user who is also performing the surgical operation, whilst the microscope is optically focussed on a focal plane over the tissue sample using the microscope objective lens 210 of the OCT scanner adapter 206 via which the OCT probe beam is emitted onto the tissue sample.

Some embodiments of the OCT scanner adapter 206 microscope accessory shown in Figures 2A and 2B include a scanning mirror assembly 310 (described in more detail below) having a compact optical design which allows h2 to be minimised to 36 mm or less.

For example, in some embodiments, and as describer later below referring to Figures 3A, 3B and 4, the OCT scanner adapter 206 comprises an ultra-compact large numerical aperture micro-electro-mechanical system, MEMS, based two-dimensional, 2D, scanning mirror assembly 310 which uses a point source 158 for determining a position of the reflective mirror surface 334 in its optical design using a position sensitive detector 160.

Some embodiments of the OCT scanner assembly using the PSD 160 are able to support very high-speed scan rates, for example, 36000 A-scans per second or higher, where an A-scan is a depth scan at a point in the tissue. Each B-scan is formed from multiple adjacent A-scans of which can be used to generate an image with depth information for the area being scanned in the form of a slice through the sample being scanned to show structures at different depths along the slice. In other words, a B-scan provides information about structures in the z-direction or depth direction along a single linear traversal of the tissue sample, for example, a linear scan along a line definable in x-y coordinates such as those shown schematically in Figure 1. Some embodiments of the OCT assembly allow very high-resolution images, for example, 400 B-scans per second, to be generated in real-time across the full field of view (FoV) being scanned, which may be for example, a 20mm x 20mm area or larger. By performing a series of B-scan sufficiently quickly and close to each other across the sample, a three-dimensional volumetric or composite scan can then be formed of the area being scanned and presented on a display 152.

The embodiment of the scanning mirror assembly shown as an optical block 310 in Figures 3A to 3B, 4, and in more detail in Figures 5A and 5B comprises various optical components which are arranged in an optical design configured to reduce the height h2 and lateral footprint of the OCT scanner adapter 206.

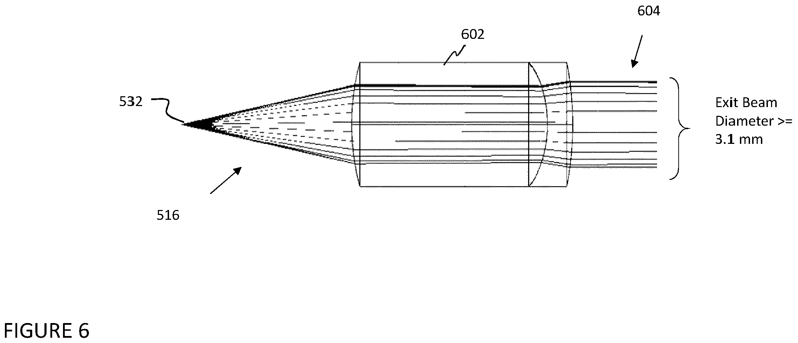

Some embodiments of the optical design of the scanning mirror assembly specify one or both of a minimum and a maximum exit beam diameters for one or more optical components. For example, the beam diameter of the OCT beam input via the optical fibre 308A when it exits the collimating lens assembly 516, shown as collimating lens 602 in Figure 6, is preferably above a threshold diameter of 3.1mm, and the beam diameter from the collimating lens may have a 3.3mm exit pupil diameter in some embodiments. Other design constraints may be dependent on the exit pupil diameter of the OCT beam from the collimating lens. For example, in some embodiments, the collimated OCT beam exits the collimating lens assembly with an exit pupil diameter of at least 3.1mm, possibly as large as 3.3mm, and have less than (about) a ½ wave (rms) wave front error.

Another exit beam diameter which is selected for is the beam diameter of the OCT beam 312 that exits from the focusing lens assembly 314 of the OCT scanner adapter 206 which then is incident on the fold mirror 316.The focussing lens 314 expands the OCT scanning or probe beam diameter to 10.6mm to set the OCT system numerical aperture and ultimately the resolution for the OCT scanner system based on the focal length of the OCT microscope objective lens 210. For the case of the 175mm working distance (not focal length) objective lens 210, the lateral resolution is 30µm, in other words, the resolution is better than 33 line pairs per millimetre. This can be contrasted with the 6 micron, 166 line pairs per mm, resolution at the intermediate image plane located at the exit of the OCT objective lens assembly510, 512.

In some embodiments, the maximum FoV which a user can set for a scan is a 20mm by 20mm area using a suitable user interface, for example, a user interface of an apparatus implementing the image processing system 148 shown in Figure 1 of the figures which includes or is connected to display 152. In some embodiments, the user interface is configured so that a user can adjust a location of an OCT scan FoV within a 25mm box although a full FoV of an OCT scan image remains a 20mm x 20mm area.

Embodiments of the disclosed technology which are used for surgical procedures and other use contexts requiring real-time image processing may use a high-dispersion configuration of the OCT system 100 with the OCT scan adapter 206.

The term real-time as used herein, refers to processing delays which are imperceptible, for example, 60ms or less, and delays of around 30ms or less are also achievable in some embodiments. The design incorporates a high angle of incidence at the scanning mirror to reduce the compound angle coupling when performing a 2D scan of a sample with both high lateral optical resolution and a telecentric image plane.

The following description of the optical design of the scanning mirror assembly 310 shown in Figures 3A, 3B, 4 and 5A incorporates a high angle of incidence at the scanning mirror reflective surface 334 to reduce the compound angle coupling when performing a 2D scan of a sample to enable the OCT scans to be performed with both a high lateral optical resolution and a telecentric image plane. Each OCT-scan comprises a large number of one-dimensional scans, A-scans, which provide depth information at a point in the area (e.g. of the sample) being scanned. Several A-scans are stacked together to create a two-dimensional image, referred to herein as a B-scan. A B-scan provides a slice through the scanned area showing depth information along the path of the A-scans. A plurality of B-scans traversing the scanned area may provide a volumetric scan in three-dimensions of the scanned area.

The scanning mirror assembly 310 includes a moveable reflective surface 334 comprising a micro-electro-mechanical system having a suitably large numerical aperture. The term "large numerical aperture" here refers to a clear aperture of the reflective surface 334 which is preferably greater than about 4mm in diameter. The term "clear aperture" means the range of angles which can be imaged via the aperture without any supports or clips or other forms of retaining elements getting in the way. The larger the diameter of the clear aperture of the scanning mirror reflective surface 334, the slower the scan rate as the probe beam then covers a larger diameter. Whilst a MEMS mirror with a clear aperture of around 7mm is already known in the art, even with optical feedback, such known MEMS mirrors with large clear apertures are not capable of scanning at an acceptable rate for real-time imaging applications such as are required for OCT when performing optical surgery. In some embodiments, the scanning mirror has a 5mm clear aperture. In some embodiments, a 4.2mm clear aperture diameter scanning mirror assembly is used which enables scans to be performed at a sufficiently high rates for the SD-OCT system shown in Figure 1 to be used for real-time surgical applications.

In OCT, the axial and lateral properties are decoupled. Lateral resolution is defined by the objective and focusing media in front of the sample. Axial properties of the interferometry are defined by the coherence properties of the OCT scanning light source and also how, after being returned from the sample, the returned OCT signal is sampled at the detector. The OCT axial resolution is dependent on the spectral bandwidth of the OCT scanning light source and the centre wavelength. Axial imaging depth defines the axial range which is covered in a B-scan. It is also defined by the maximum fringe frequency that can be detected as the maximum frequency of an interference spectrum is what decodes the maximum depth scanned.

An A-scan, is an amplitude depth scan along one-dimension, usually referred to as the z-axis, through a sample, a B-scan is a lateral scan in two-dimensions across a sample formed by a series of A-scans. In other words, for each sample point, the spectrally dependant interferometric fringe pattern created by the back reflection from the reference mirror of the OCT interferometer and the back reflection from the sample is recorded as an A-scan. Multiple A-scans are made to generate other scans such as B-scans which allow a complete depth profile of the sample reflectivity at the beam position to be generated.

In some embodiments, the open aperture diameter is 4.2mm or above.

In some embodiments, the OCT scanner adapter 206 includes a high speed OCT MEMS based mirror scanning assembly 310 which uses a position sensitive detector system 160 to provide a control loop feedback for controlling the positioning of the OCT beam during the scan. The control loop feedback has a technical benefit in that it allows the OCT scanner to generate more B-scans per second of the object of interest being scanned. In other words, the control feedback loop provided in some embodiments of the disclosed technology allows suppression of ringing behaviour and resonant behaviour caused by a step change in driving voltage at the end of a scan line.

### EXAMPLE(S) OF OCT MICROSCOPE ADAPTER DESIGN

Figure 3A shows schematically an example embodiment of a MEMS microscope OCT scanner adapter 206 according to the disclosed technology which is suitable for mounting to the under-carriage of the microscope 200 shown in Figures 2A an 2B as it has been configured with optical components which seek to optimally reduce the lateral and vertical footprints whilst maintaining suitable optical quality characteristics for OCT applications.

In Figure 3A, the illustrated example embodiment of the OCT scanner adapter 206 comprises several components contained within or mounted on an adapter housing 208. The adapter housing 208 comprises the data/power port, 212 for example an Ethernet power over Ethernet port or high-speed USB port or the like.

An optical port 214 is also provided for OCT scanning light to be input and output from the OCT scanner adapter 206. An optical fibre 308a connected to the optical port 214 feeds in OCT light from the coupler 104 shown in the OCT scanning system 100 of Figure 1 to the scanning mirror assembly optical block 310 shown in Figure 3A via optical fibre connector 308. The returned OCT light travels back along via optical fibre 308a to the coupler 104 shown in Figure 1. The optical fibre 308a is accordingly part of the optical path 105a shown in the OCT scanning system 100 of Figure 1 via which OCT probe light illuminates the sample to be scanned and via which OCT light returned from the sample is output towards the coupler 104 of the OCT scanning system 100 shown in Figure 1. The optical fibre 308a has a suitable numerical aperture, preferably 0.14, to allow OCT light in the near-infra red region to propagate along it in a single mode.

OCT Light from the OCT light source 102 follows an optical path 101a along the illumination arm 101 to the coupler 104 and then takes optical path 105a along the probe arm 105 in Figure 1 of which the optical fibre 308a forms part. The OCT light following the optical fibre 308a is injected into the scanning mirror assembly optical block 310 via OCT data connection fibre connector 308 and then follows the OCT arm 518 (see Figure 5A) of the MEMS scanning mirror assembly 310.

In some embodiments, the optical fibre connector 308 via which OCT light is input to the MEMS mirror block 310 is a fibre connector to angle polished connector.

The optical block housing the MEMS scanning mirror assembly 310 also houses the optical components of the optical angular displacement mirror position measurement system 156 for the scanning mirror assembly, shown in Figure 1 as mirror position measurement system 156. The controller 162 (see Figure 1, not shown in Figure 3A) is used for adjusting the reflective surface 334 of the scanning mirror 112 shown in Figure 1 using a mirror mover mechanism (not shown) of the MEMS scanning mirror assembly 310. The controller 164 may be implemented within the OCT scanner adapter 206 or provided remotely, in which case control signals may pass to the mirror mover in the MEMS scanning mirror assembly 310 via the data port 212 of the OCT scanner adapter 206.

The same mirror reflective surface 334 in the scanning mirror assembly housed in optical block 310 reflects both the input OCT beam and a mirror positioning reference beam from a different source (see Figure 5A described below for more detail) in some embodiments. As would be apparent to anyone of ordinary skill in the art, however, it is possible for separate mirrors mounted on the same tilt-axis to be used in other embodiments, providing this is done in a manner that does not adversely affect the stack height h2 of the OCT scanner adapter 206.

The OCT light following the optical path 105a received via OCT data connection fibre 308 is reflected in a different optical plane at the reflective surface 334 to the optical plane at which light from the light source of the angular displacement mirror measurement system 156 is reflected.

The reflected OCT beam then follows an optical path through the OCT scanner adapter 206 from which it emerges via the microscope objective lens 210 to probe the object of interest, for example, a tissue sample such as the in-vivo eye tissue shown schematically in Figure 1. Other types of objects of interest may range from tissue samples for ophthalmology and areas such as dermatology, dentistry, angiography, cardiology, as well as other tissue samples for diagnostics of diseases including cancer.

The OCT light which is reflected, back-scattered or otherwise returned from structures within the tissue sample then follows a return path 105a back through the scanning mirror assembly of the optical block 310 and along optical fibre 308a. The returned OCT light then exits the OCT scanner adapter 206 via optical port 214 and is fed into the OCT system 100 where it is combined and interferes with light returned from reference arm 103 at coupler 104. The resulting interference pattern is detected in the OCT system 100 of Figure 1 by a spectrometer 136 which generates image data which can then be image processed in order to obtain a tomogram image indicating the scanned structures in the tissue located within the scan FoV.

In the example embodiment of the OCT adapter 206 shown in Figure 3A, the OCT probe beam 312 is output from the optical block which houses the MEMS OCT scanning mirror assembly 310 and travels towards a fold mirror 316 which lifts the OCT beam by a minimal amount out of its optical plane towards beam splitter 318. The beam splitter 318 reflects the incident OCT beam towards the objective lens assembly 210 of the OCT scanner adapter 206 which also acts as an objective lens for the microscope optics housed in microscope 200 when the OCT scanner adapter 206 is attached the microscope 200. The OCT probe beam emerges from the objective lens 210 as a telecentric beam focussed on focal plane 154 in the tissue being scanned, which as shown lies in the x-y plane shown schematically in Figure 1. The resulting returned OCT light may be used to generate an OCT A-scan which provides depth information orthogonal to the focal plane 154, in other words, in the z-direction as shown in Figure 1. Movement of the reflective mirror surface 334 moves the location of the telecentric beam across the focal plane 154 in the area being scanned 116 and allows OCT B-scan images to be generated.

In Figure 3A, the OCT scanner adapter 206 is configured so that the objective lens 210 can be used as an objective lens by microscope optics as well as by the OCT scanner system 100. A light-proof gasket 320 is provided around the aperture formed in the OCT scanner adapter 206 via which the OCT objective lens 210 is aligned with and extends the optical channel formed by the microscope optics of microscope 200.

The configuration of the fold mirror 316, the beam splitter 318, and the objective lens assembly 210 of the OCT scanner adapter 206 are collectively designed so that OCT beam is lifted out of the plane it follows through the scanning mirror assembly by only a small amount, in order to be able to exit via objective lens 210. The amount of lift required is affected by the tilt-angles for the beam splitter and the fold mirror and beam entry geometry. The additional height h2 that the OCT scanner adapter 206 adds to the height of the microscope is accordingly also minimised by using this optical design. For example, in some embodiments the OCT scanner adapter housing 302 adds 40mm or less to the overall height h1 of the microscope housing 202. In some embodiments, the additional height h2 is 36mm or less. This can be achieved with a lift of, or around, 27mm in some embodiments using suitable tilt-angles for the beam-splitter and fold mirror.

In some embodiments, and as shown in the example embodiment of Figure 3A, the OCT probe beam 312 exits the optical block housing the scanning mirror assembly and travels through free space first to a focussing lens assembly 314 which allows the focal plane of the image being scanned to be adjusted. This adjusts the focus of the scan at different depths. The focussing lens assembly 314 is driven by a motor 326 and also comprises a travel limiter or stop 324. In some embodiments, the focussing mechanism provided by the focussing lens assembly 314 of the adapter can be adjusted to control the OCT focal plane with a ±30mm range which allowing a range of depths in the sample to be focussed on for a scan. The OCT focal plane can be adjusted over the +/-30mm range and may be optimized for the best SNR during initial image acquisition. This is different and should not to be confused with the technique of shifting the focus at the A-scan rate which is used to extend the depth of focus within the sample. The focusing lens assembly 314 moves more slowly than is required for the A-scan sampling and unless the system detects a large motion in the sample it will not be adjusted without user intervention.

In some embodiments, and as shown in Figure 3A, the OCT scanner adapter 206 is attached to the microscope 200, using fixations, for example, screws, which are provided in recesses of mounts 328a and 328b and which can be extended out of the mounts 328a, 328b into the undercarriage of the microscope 200 into corresponding receiving apertures or holes, preferably threaded holes in the undercarriage of the microscope 200 so as to fixedly engage the OCT scanner adapter 206 with the microscope. In some embodiments where the OCT scanner adapter 206 functions as a microscope accessory the OCT scanner adapter 206 may also comprise receiving apertures or holes at corresponding locations in its base to the locations of receiving holes or apertures in the undercarriage of the microscope 200. By having the same or similar fixation locations in the base of the OCT scanner adapter microscope accessory 206 as the fixation locations in the microscope undercarriage, different types of microscope accessories which would otherwise be attached to the undercarriage of the microscope 200 can instead be attached to the undercarriage of the OCT adapter. In other words, in some embodiments the OCT scanner adapter 206 is configured to attach to the undercarriage of the microscope as a microscope optics accessory. Some embodiments, of the OCT scanner adapter microscope accessory 206 allow the OCT scanner adapter accessory 206 to have another microscope accessory attached to the base of the OCT scanner adapter.

Figure 3B shows an alternative view of the OCT scanner adapter 206 of Figure 3A. In Figure 3B, however, the location of the optical source or emitter 158 of the mirror positioning light beam 400, shown in Figure 4, which illuminates the scanning mirror is more visible along with as is the location of a position sensitive detector, PSD, 160 of the optical angular displacement measurement system 156 in the optical block 310.

Also shown schematically in Figure 3B is an example angle of incidence θ of the mirror positioning illumination beam 400, shown in Figure 4, at the reflective surface 334 the MEMS scanning mirror which after reflection forms the mirror positioning reference beam which travels towards the PSD 160.

It will be appreciated that the angle of incidence and the locations of the beam paths shown in the drawings are for illustrative purposes only and not to scale.

The design of the MEMS scanning mirror assembly is configured so that the illuminating mirror positioning light beam 400, shown in Figure 4, is reflected in a different optical plane by a reflective surface 334 of the scanning mirror to form a reference beam 402 which passes along a positioning reference arm of the OCT scanning mirror assembly 310 from the optical plane in which the same reflective surface 334 reflects the incident OCT scanning or probe beam 312, shown in Figures 4 & 5A. The scanning mirror assembly is also configured so that returned positioning light is reflected by the mirror in another optical plane different from the optical plane of reflection of the OCT outwards and returned beams and different from the optical plane in which the incident mirror positioning beam is reflected so that it causes minimal interference with either OCT beams or the incident mirror positioning beam or the optical source for the mirror positioning beam.

Figure 4 of the accompanying drawings, this shows schematically an enlarged view of the OCT scanner adapter 206 of Figures 3A and 3B. In Figure 4, the mirror position illuminating beam 400 (shown as a dash dash line) from the point light source 158 is incident at the reflective surface 334 of the OCT scanning mirror 500, shown in Figure 5A, with an angle of incidence, AOI, shown as θ. The mirror position reflected beam 402 (shown as a dash dot dash line in Figure 4) is reflected towards the PSD 160 where it is detected. For the sake of clarity, returned light from the incident beam at PSD 160 is not shown in Figure 4.

The OCT scanner optical components shown in Figures 3A to 4 are arranged so that OCT light emerging from the optical plane of the scanning mirror assembly is lifted by only a small amount out of that optical plane by folding mirror 316 towards the beam splitter 318. Beam splitter 318 allows light via the microscope optics to be transmitted and returned to the microscope optics whilst also reflecting the OCT probe beam to the same focal plane 154 as the microscope light. By optimally positioning the beam-splitter and the fold mirror relative to the objective lens 210, it is possible to reduce the height the OCT probe beam must be lifted by the fold mirror before it is reflected by the beam splitter 318 out through the objective lens 210.

In some embodiments, the fold mirror lifts the OCT beam by 27mm mm out of the optical plane of the scanning mirror assembly.

### EXAMPLE(S) OF SCANNING MIRROR ASSEMBLY OPTICAL DESIGN

Figure 5A of the accompanying drawings shows schematically an example of an optical design for a two-dimensional, 2D, scanning mirror assembly such as, for example, the 2D scanning mirror assembly housed in the optical block 310 shown in Figures 3A, 3B and 4.

The optical design of the 2D scanning mirror assembly is suitable for use in other types of OCT scanners such as OCT scanner 164 in Figure 1 as well as the OCT scanner adapter 206. The scanning mirror assembly 310 shown in Figure 5A has an optical design which may be used for other applications than OCT which use scanning light requiring mirror positioning.

In other words, the 2D scanning mirror assembly optical design of Figure 5A does not need to be limited to OCT applications or apparatus such as those shown in Figures 1-4 of the accompanying drawings in all of its embodiments. It may be beneficially implemented in any other types of light scanning apparatus where a compact lateral optical plane is beneficial.

Some example embodiments of the micro-electro-mechanical system, MEMS, two-dimensional scanning mirror assembly 310 shown in Figure 5A have an optical design comprising a moveable MEMS scanning mirror having a reflective surface 334, a connector 308 to a point light source for a scanning light beam, for example, as shown an optical fibre 308a connected via an optical fibre connector 308 and the end of the optical fibre 308a (see Figure 5b) then acts as the point light source for the light beam.

The scanning mirror assembly optics also comprises a collimating lens assembly 516 for the light introduced via the connector 308. Collimating lens assembly 516 is configured to output light from the point light source with an exit beam diameter above a threshold exit beam diameter towards the reflective surface suitable for a desired scanning application. After reflection at the reflective surface 334 of the scanning mirror 112, the scanning light beam passes via an objective lens assembly 510, 512 to exit the scanning mirror assembly.

The reflective surface 334 is configured to reflect an incident collimated light beam to form a scanning beam, for example, an OCT probe beam if the point light source provides OCT light, which exits the mirror assembly via an objective lens 510 and field lens 512 (collectively also referred to as the objective lens assembly 510, 512) as a telecentric beam 312 towards a telecentric image plane 154. The optical design of the components in the scanning mirror assembly is configured to ensure the scanning beam is able to perform a scan with a resolution better than a resolution threshold.

The optics of the scanning mirror assembly are configured to provide a total track length, L, for the path from the point light source, for example, from the end-face of the optical fibre or fibre ferrule (see also Figure 5B), to the telecentric image plane (700) of less than about 40mm to help keep the lateral dimensions X of the scanning mirror assembly small enough to allow the OCT scanner housing 308 to be below a desired lateral footprint in its design. For example, as shown in Figure 5, the width X of the scanning mirror assembly is preferably below 41m, for example, it may be 40.6mm or less in some embodiments. The optical design is also configured to keep the depth Y as shown in Figure 5A also as small as possible, for example, Y may be 35 mm or less, and may be as short as 34.5mm or less in some optical designs.

The total track length, L, is accordingly kept as short as the optical design layout permits to reduce the lateral and depth footprints X and Y to the smallest possible in some embodiments so that the housing of a scanner comprising the scanning mirror assembly 310 can be similarly provided with a small footprint..

By keeping the lateral footprint X as small as possible, side access to the area being scanned is improved which is particularly beneficial when the scanning mirror assembly 310 is a scanning mirror assembly for an OCT scanner adapter 206 which is a surgical microscope accessory as this may improve access to the area being surgically operated on whilst the microscope it is attached to is in use.

In some embodiments of the scanning mirror assembly, the threshold for the exit beam diameter from the collimating lens 516 is at least 3 mm, and preferably at least 3.1 mm. By having an exit beam diameter of at least 3.1 mm, the scanner benefits from a better lateral resolution than a small exit beam diameter allow

In some embodiments of the scanning mirror assembly, the threshold for the telecentric beam resolution at the telecentric image plane 700 is better than 6 microns. In other words, the scan image can resolve features in the sample being scanned which are smaller than 6 microns.

In some embodiments, the scanning mirror 112 may be moved, for example by a controller 162, The scanning mirror assembly may be configured in some embodiments to move about its optical axis and scan over +/- 5 degrees in some embodiments.

In some embodiments of the scanning mirror assembly, the numerical aperture of the optical fibre and the focal length of the collimating lens together determine the a suitable threshold for the exit beam diameter of the collimated beam from the collimating lens to at least 3.1mm into achieve the designed resolution at the focal plane. The combination of focal lengths of a scanning mirror objective lens and a scanning mirror field lens via which the probe beam exits the mirror assembly determines the total track length, L, which is preferably less than 40mm or thereabouts.

In some embodiments, for example, where the scanning mirror assembly is being used for OCT purposes, the optical fibre has a numerical aperture of 0.14. The optical fibre which feeds in light to the scanning mirror assembly by acting as a point light source may have another suitable numerical aperture value in other embodiments of the scanning mirror assembly providing the numerical aperture allows sufficient light to be fed into the scanning mirror assembly for another context of use along a single mode optical fibre 308a.

In some embodiments of the scanning mirror assembly, the objective lens 510 comprises a F2.7 Biconvex doublet lens and the field lens 512 comprises a F19 positive/negative meniscus doublet field lens.

In some embodiments of the scanning mirror assembly, the optical path difference, OPD, of the telecentric probe beam output by the scanning mirror assembly has a radius of curvature is greater than 100mm.

In some embodiments of the scanning mirror assembly, the telecentric beam is telecentric to better than an incident angle of 0.03 degrees at the telecentric image plane.

In some embodiments of the MEMS scanning mirror assembly, the reflective surface 334 of the MEMS scanning mirror comprises a large-aperture gold-coated silicon mirror bonded to an underlying mechanical structure.

The embodiment of the scanning mirror assembly 310 design illustrated schematically in Figure 5A may be implemented as an optical block in an OCT scanning system such as the OCT scanning system 100 shown in Figure 1. For example, in some embodiments, the scanning mirror assembly is implemented as an optical block having the X,Y footprint shown in Figure 5A in a compact OCT scanner adapter 206 for a microscope, such as the optical block which houses the scanning mirror assembly 310 shown in Figures 3A, 3Band 4.

As mentioned above, however, the scanning mirror assembly 310 shown in Figures 5A and 5B has an optical design which can be used in a range of different use contexts in other types of scanner systems. In some embodiments, the mirror assembly shown in figure 5A and 5B is provided as scanning mirror assembly for an OCT apparatus such as that shown in Figures 3A to 3B and receives light injected by optical fibre 308a. In other embodiments a different point light source may be used instead of the optical fibre 308a which acts a point light source for an OCT light beam as shown in Figures 3A, 3B, 4, 5A and 5B.

In some embodiments, the mirror assembly 310 may be provided in an OCT scanner adapter 206 which is used as an OCT scanning accessory for a microscope. 200 The microscope may comprise a surgical microscope in some embodiments and the scanning mirror assembly 310 may be used to generate OCT scans of a sample tissue area being surgically operated on at a sufficiently high rate to allow live OCT tomographs to be generated of the sample tissue area whilst the surgical operation is on-going.

In some embodiments, the SD-OCT scanning system shown in Figure 1 includes a. OCT scanner adapter 206 comprising a scanning mirror assembly 112, 310 having the optical design shown in Figures 5A and 5B and as described herein.

In some embodiments, the scanning mirror assembly 310 is configured so that OCT light returned from the sample along the OCT probe arm 105 has a lateral optical resolution equal or higher than 6µm, in other words, the resolution is better than 166 line pairs per mm.

In some embodiments, the scanning mirror assembly 310 comprises a MEMS 2-D scanning mirror assembly which includes at least: a moveable MEMS scanning mirror having a reflective surface 334, an optical fibre 308a connected via an optical fibre connector 308 and configured to act as a point light source for an OCT beam illuminating the reflective surface 334, a collimating lens assembly 516 configured to output OCT light from the point light source with an exit beam diameter of at least 3.1mm towards the reflective surface 334. The reflective surface 334 is configured to reflect both an incident collimated OCT light beam to form an OCT probe beam and a mirror positioning reference beam. The OCT probe exits the mirror assembly as a telecentric beam towards a telecentric image plane with a resolution of at most 6 microns. The optics of the scanning mirror assembly 310 are configured to provide a total track length, L, from a) an end-face of a fibre ferrule providing the point light source inserted in the optical fibre connector to b) the telecentric image plane of less than 40mm and preferably less than 36mm in some embodiments. An objective lens assembly 510, 512 is provided in a probe arm of the scanning mirror assembly to focus the telecentric OCT beam via the OCT scanner (microscope) lens 114, 210 in some embodiments.

The scanning mirror assembly 310 has an optical design which includes the reflective surface 334 of the MEMS mirror being configured so that an incident mirror positioning beam is reflected in a separate optical plane to the optical plane in which an incident OCT scanning beam is reflected. In this manner, the scanning mirror assembly may be also used with a mirror positioning system such as an angular tilt mirror positioning system shown schematically in Figure 1 of the drawings.

As mentioned above, some embodiments of the MEMS based scanning mirror assembly shown in Figures 5A and 5B and described herein are implemented in an OCT scanner 206 such as that shown in Figures 3A,3B and 4, as part of the SD-OCT scanning system shown in Figure 1. Some embodiments of the disclosed technology accordingly comprise a OCT scanner system 100 comprising the OCT scanner 206 including a micro-electro-mechanical system, MEMS, two-dimensional scanning mirror assembly 310 having a compact optical design according to the disclosed technology.

In some embodiments of the MEMS scanning mirror assembly 310, the scanning mirror 112 is mounted on an underlying mechanical structure or support 500 as shown in Figure 5A which provides a mirror moving mechanism to allow the mirror surface 334 to pivot about its optical axis under the control of controller 162.

In some embodiments, the reflective surface 334 of the MEMS scanning mirror assembly comprises a large-aperture gold-coated silicon mirror bonded to the underlying mechanical structure 500.

The angular displacement measurement system 156 shown in Figure 1 is implemented in the embodiment of the MEMS mirror assembly of Figure 5A, by a light source 158 which comprises a suitable optical point source, for example, a laser diode 502. The optical point source generates a light beam, referred to herein as a mirror positioning light beam 400 (shown by the dashed line in Figure 5A) which passes through a collimating lens 503 such that a collimated mirror positioning beam 400 is incident on the scanning mirror surface 334 with an angle of incidence θ.

The angular displacement measurement system 156 is used to determine the angular position of the MEMS, scanning mirror assembly 310 relative to the incident mirror positioning beam 400 as this allows the mirror position for an incident light beam to be determined when performing a scan and adjusted as the scan progresses. The OCT scan (e.g. a B-scan or volumetric scan) is performed by moving the mirror using the controller 164 in accordance with any scan parameters for a particular scan configuration (these can be input by a user and/or automatically determined for a particular type of scan in some embodiments).

The position of the moveable MEMS mirror surface 334 may be controlled in some embodiments using a suitable angular position controller (not shown in Figure 5A) using closed loop control based on the feedback from a position sensitive detector 160 which detects the reflected mirror positioning beam 402.

In some embodiments, the scanning mirror assembly 310 described above with reference to Figures 3A, 3B, 4, 5A and 5B includes the position sensitive detector 160 which is configured to send feedback mirror position data to a controller 162 configured control the position of the MEMS scanning mirror surface when a scan is performed. However, in some embodiments of the compact OCT scanning mirror assembly 310 of the drawings, the controller is housed remotely. For example, it may be housed elsewhere within the OCT scanner adapter 206 in some embodiments. Alternatively it may be housed with other system components of the OCT scanner system 100 or hosted on a different platform having a user interface to allow the input of scan parameters in some embodiments. Control signals may be sent from a remote controller 162 via a suitable data connection such as data port such as 212 in some embodiments.

In some embodiments, the mirror positioning light source illuminates the reflective surface of the mirror assembly for the optical mirror position feedback channel with an angle of incidence, θ, above 62 degrees, preferably 67.5 degrees, from the normal to the plane of the reflective mirror surface 334.

In some embodiments, the OCT light source illuminates the reflective surface of the mirror assembly for the OCT light channel with an angle of incidence, θ, below 28 degrees, preferably 22.5 degrees, from the normal to the plane of the reflective mirror surface 334.

In some embodiments, the minimum usable aperture at the reflective mirror surface is at least 4mm, which is particularly useful when the mirror assembly is incorporated in OCT scanner apparatus, such as the compact OCT scanner 206 microscope accessory used for surgical applications.

In some embodiments of the disclosed technology, the OCT apparatus may use closed loop feedback for controlling the scanning mirror position in some embodiments. The use of closed loop feedback may be useful in embodiments where a high scan rate is required such as where a live video or other form of image sequence of OCT scans is required. The use of closed loop feedback supports OCT scans being generated at a high-rate with low lag for time-sensitive applications, such as when OCT scans are provided to guide surgical procedures as it allows the mirror to be moved fast enough and precisely enough to achieve high scan rates and/or high scan resolutions (in other words, high OCT image B-scan or volumetric scan resolutions). In some embodiments, however, an open loop control may be provided.

The disclosed technology seeks to address at least some of the design constraints which are present when designing OCT systems for surgical microscopes. For example, one design constraint is that smaller diameter scanning mirror surfaces are better suited to achieving higher scan rates. The numerical aperture relates to resolution. A clear aperture, i.e., mirror diameter, relates to scan size in that the underlying mechanical structure of the MEMS is the same so a small diameter mirror can such as 2mm diameter can tilt farther before hitting the MEMS base (up to +/-7 degrees) where-as large diameter mirrors such as 7.5mm diameter can only tilt +/-1.5 degrees before hitting the base. This means that whilst a smaller diameter mirrors could be used and scan a larger area, this would be at the cost of resolution.

In some embodiments, the threshold for the exit beam diameter of the OCT light beam is based on a numerical aperture of the optical fibre and the focal length of the collimating lens assembly.

In some embodiments, the two-dimensional scanning mirror assembly is configured to reflect a mirror positioning beam of light (400) incident at the reflective surface (334) in a first optical plane towards a position sensitive detector (160) configured to generate information on an angle of tilt of the scanning mirror reflective surface (334

In some embodiments of the optical angular displacement measurement system 156 of the scanning mirror assembly 310 shown in Figure 5, the mirror positioning light from light source, 158 is collimated first by a suitable collimating lens assemble 503 to form a collimated illuminating light beam 400 which is incident at the reflective mirror surface 334. The collimated illuminating light beam 400 (represented schematically by the short dash dash line in Figures 3B, 4 and 5A) is then incident on the reflective MEMS mirror surface 334 with an AOI = θ and is reflected to form a mirror position reference beam 402 (shown by the longer dash dot dash line in Figures 3B, 4 and 5A) which travels along a mirror positioning reference arm 501 of the scanning mirror assembly towards PSD 160 via a PSD lens assembly 504 and, in some embodiments, via an optional neutral density filter 506.

The mirror positioning beam 400 may, however, be reflected back or otherwise returned by the PSD 160 towards the reflective surface 334 of the MEMS mirror (the reflected beam is not shown in Figure 5A). This is unwanted as such returned light can contaminate the illuminating positioning beam and/or the input OCT light beam. Other issues with stray light reflectance in the mirror position detector system include the detected spot position being wrong if there is any stray light on the PSD 160, the diode behaviour may change if reflected light enters the diode cavity, and this can cause intensity fluctuations in the position detector beam which the PSD will detect as changes in position.

To prevent returned reflective elements of the positioning beam being reflected by the MEMS mirror assembly 310, some embodiments of the disclosed technology include additional components such as a light trap. The light trap is suitably configured and located suitably to reduce any reflected mirror positioning reference beam light from re-entering the emitter for the mirror positioning beam and/or contaminating the returned probe beam 312 before it reaches the interferometer.

As mentioned above, some embodiments of the MEMS based scanning mirror assembly shown in Figures 5A and 5B have reflective surface(s) is (are) is designed so that OCT light input via the OCT light coupler 308 is reflected from another region of the MEMS mirror surface 334 that the mirror positioning reference beam 402 and the OCT scanning or probe beam are reflected in different optical planes.

The OCT scanning or probe beam reflected by the MEMS mirror surface 334 of the scanning mirror assembly 310 after reflection passes along an optical path through an OCT objective lens 510 and an OCT field lens assembly 512 which outputs the OCT beam as a telecentric beam into free space towards fold mirror 316. As shown in the embodiments of Figures 3A and 3B, and 4, before the beam is incident on fold mirror 318 which lifts the beam out of the optical plane of the scanning mirror assembly it passes via focussing lens assembly 314. In some embodiments this allows the OCT focal plane to be focussed with a +/- 30mm range, in other words, a range of different depths can be focussed on in the scan area. However the focussing lens optics may be omitted in some embodiments of the OCT scanner.

The fold mirror 316 lifts the OCT scanning (or probe) beam out of the plane of its optical path through the scanning mirror assembly by reflecting the incident OCT scanning or probe beam towards a beam-splitter 318. The beam-splitter reflects the OCT scanning or probe beam out of the OCT scanner adapter 206 microscope objective lens 210 towards a focal plane 154 for scanning a tissue or similar object of interest, which may be an in-vivo tissue sample or in-vitro sample. The beam-splitter 318 also allows the OCT illuminated area being scanned to be viewed via microscope optics housed in the microscope 200.

In some embodiments, and as shown in Figures 3Aand 3B, the OCT probe beam 312 is input to the optical block by traveling along optical path 105a within optical fibre 308a and enters the scanning mirror optical block 310 via OCT data connection fibre input 308. The OCT scanning or probe beam 520 then passes via collimating lens 516 towards the scanning mirror reflective surface 334. The mirror surface 334 reflects the OCT beam via a probe arm 508 out of the optical block containing the scanning mirror assembly 310 at which point it travels in free space towards the fold mirror 316.

As shown in the embodiment of the OCT adapter shown in Figures 3A and 3B and 4, the OCT scanning or probe beam 312 is focussed before it reaches the fold mirror 316 by passing through a focusing lens assembly 314. The focussing lens assembly is driven by a motor 336 which adjusts the position of the focussing optics to allows a range of focal depths to be achieved when performing a scan. In some embodiments, the focal range can vary from +/- 30mm.

The returned OCT light is reflected via the MEMS scanning mirror surface 334 back along the OCT arm 518 of the scanning mirror assembly 310 back towards the coupler of the OCT system 100 shown in Figure 1.

In the scanning mirror assembly 310, the input OCT light beam is input via the optical fibre connector 308 from the end face 532 of optical fibre 308a passes at the optical fibre ferrule 530 (see also Figure 5B) through an OCT collimating lens 516 towards the scanning mirror assembly. The track length, in other words the measurable physical distance of the path taken from the end-face 532 to the surface of the scanning mirror is shown in Figures 5A and 5B as L1.

Figure 5A also shows the track length L2 from the scanning mirror surface to the telecentric image plane 700. The total track length L = L1 and L2 is preferably less than or equal to a track length design threshold of 40mm.

Figure 5B is an enlarged view of Figure 5A showing more clearly the location of the optical fibre ferrule 532 and optical fibre end-face 530 at which the optical fibre 308a acting as a point light source injects OCT light into the mirror scanning system 310. The OCT light passes from the end face 532 of the fibre to collimating lens 516 and the collimated illuminating OCT beam is then incident at the reflective surface 334 of the MEMS scanning mirror which reflects it towards the OCT probe arm 105 (as shown in Figure 1) or 508 as shown in Figure 5A.

In the return direction, which is not shown in Figures 5A or 5B for clarity, returned OCT light passes along the OCT arm 518 (see also the description of Figure 6) and through the collimating lens 516 in the other direction before travelling out via the OCT data connection fibre 308 along optical fibre 308a before leaving the OCT scanner adapter 206 via optical port 214.

In some embodiments, the OCT scanner is implemented using an OTS (off the shelf) MEMS (microelectromechanical system) in which the MEMS scanning mirror reflective surface 334 is provided by large aperture protected gold coated silicon mirror bonded to the underlying mechanical structure 500 of the optical block 310. The OCT scanner 206 formed by such a design provides a simplified and miniaturized optical system which has the same optical performance as much larger galvanometer scanning mirror type systems known in the art for use in intraoperative OCT systems.

In some embodiments, the optical block design of the OCT MEMS mirror assembly 310 includes a 2D scanning mirror assembly and a complimentary optical angular displacement measurement system 156 for measuring the position of the MEMS mirror system.

The mirror positioning system which measures the angular displacement of the scanning mirror reflective surface 334 comprises a mirror positioning light source 158 and a position sensitive detector, PSD, 160. The PSD may comprises a PSD lens assembly 504 and a neutral density filter 506 as well as the PSD 160. An example of a suitable PSD detector is a Hamamastu S5991 4mm x 4mm active area position sensitive detector..

In some embodiments, the angular optical displacement measurement system 156 is provided in the same optical block as the MEMS scanning mirror assembly 310. The optical angular displacement measurement system 156 is used in some embodiments to provide closed loop control of the MEMS scanning mirror position. The closed loop control can be provided by measuring the angle of incidence θ using the PSD 160 and providing information indicating the mirror position derived from this to a controller which allows the controller to more accurately control the tilt angle of the scanning mirror reflective surface 334 as a scan is performed.

This closed loop feedback can allow much high B scan rates to be performed. For example, at least 400 B-scans per second can be achieved as a maximum scan rate at full angular deflection in relation to the maximum field of view, FoV using closed loop control for a 4.2mm diameter clear aperture mirror 112.

In embodiments without closed loop control i.e., open loop scanning, a low pass filter may be used to prevent the MEMS scanning mirror moving device from reaching its natural frequency excitation state where the MEMS scanning mirror moving device may become resonate by uncontrolled oscillation (which in turn can damage the MEMS scanning mirror moving device). In embodiments where open loop scanning is implemented, the maximum scan rate may be approximately 50 B-scans per second which can be contrasted with the rates achievable by closed loop control. With closed loop control in some embodiments, the scan rates which can be achieved using an example embodiment of the MEMs scanning mirror assembly 310 according to the disclosed technology is approximately 400Hz or higher.

In some embodiments, the optical components of the MEMS scanning mirror assembly 310 are configured collectively provide a predetermined system numerical aperture for a desired system optical resolution through the microscope objective lens 210. In other words, in some embodiments, the MEMS scanning mirror system components are suitably configured to enable the diameter of the collimated OCT beam 312 output along OCT data connection fibre 308 to match a desired minimum system optical resolution after it has passed out through the microscope objective lens 210.

In some embodiments, all air to glass interfaces within the OCT scanner adapter 206 are designed with convex surfaces to minimize any back reflections from the OCT beam as it propagates through the optical system.

Figure 6 shows an example embodiment of an OCT collimator lens, also referred to herein as an OCT collimator lens assembly, 516 such as the collimator lens 516 shown in the OCT arm 518 of the optical block including the scanning mirror assembly 310 shown in Figure 5 of the drawings. The OCT collimator lens 516 is provided along the OCT arm 518 of the scanning mirror assembly optical block 310. In Figure 6, the OCT light fed into the collimating lens assembly 602 via the optical fibre ferrule end 532 emerges as a collimated OCT output beam 604 having a collimated beam diameter of at least 3.1mm. The collimated OCT beam then travels towards and is reflected from the scanning mirror reflective surface 334. Returned OCT light follows the reverse path through the scanning mirror assembly and is focussed via the collimating beam towards the end of the optical fibre 308a which gathers the returned light and the returned OCT light can then propagates back towards a coupler 104 of an interferometer system such as OCT system 100 shown in Figure 1.

A suitable example of an OCT collimator lens 516 which may be used in some embodiments of the disclosed technology is a F3.2 Biconvex doublet lens. Such a lens has a thick crown glass section which reduces the curvature radii of the lens surfaces and consequently improves colour performance. In some example embodiments, the collimator lens has 10mm focal length with 100 micron depth of focus which allows for good mechanical focal stability. In some embodiments the OCT collimator lens provides an exit beam which has a 3.1mm diameter collimated beam (exit pupil diameter) with < % wave (root mean square, rms) wave front error.

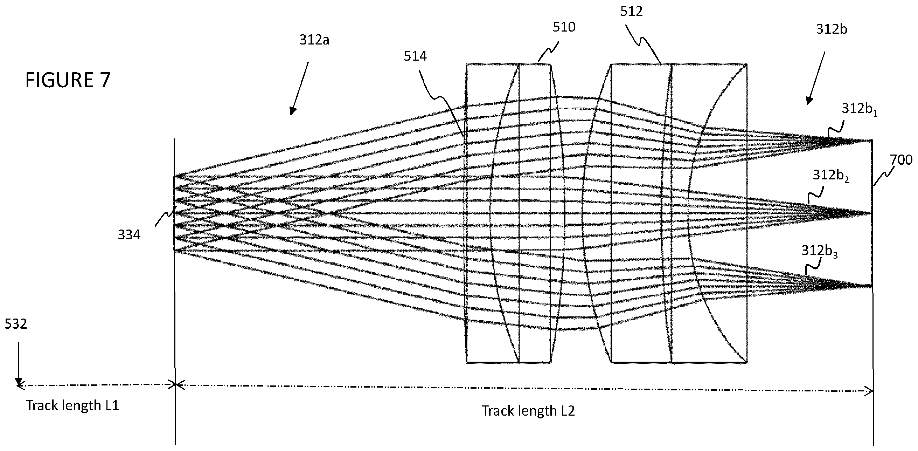

Figure 7 shows an example of an OCT objective lens assembly 510, 512 in which an OCT beam 312 comprises light 312a at a range of wavelengths, for example, a range of wavelengths over a near-infrared part of the optical spectrum.

The OCT light is reflected from the reflective surface 334 of the scanning mirror assembly is focused first by the OCT objective lens 510, and then by field lens 512 before emerging as a telecentric beam 312b. A plurality of angle dependant telecentric beams 312b_{1,2,3} are shown in Figure 7 focused on telecentric image plane 700, where each beam 312b_{1,2,3} represents where OCT beam 312b emerges at a particular scan angle, in other words, the telecentric exit beams 312b₁, 312b₂, 312b₃ are sequential beams generated as a B-scan progresses.

The OCT beam 312 deflected from the mirror surface passes, in other words, through an OCT objective lens assembly 510 (which also comprises field lens 512 in some embodiments) designed so that all scan angles of the light 312b which forms the OCT beam 312, which are shown schematically as OCT exit beams 312b₁, 312b₂, and 312b₃ in Figure 7, exit normal to the intermediate image plane and are therefore telecentric.

In some embodiments, all air to glass interface surfaces such as 514 are convex to eliminate back reflection artefacts in the OCT image. The OCT objective lens assembly illustrated in Figure 7 accordingly converts an angular input OCT scanning or probe beam 312a reflected from the MEMS scanning mirror surface 334 to a telecentric OCT scanning or probe beam 312b (or rather one of beams 312b_{1,2,3}), which is then output into free-space in some embodiments. The telecentric OCT scanning or probe beam 312b is in some embodiments first focused using a focusing lens assembly 314 before it is lifted by folding mirror 316 towards beam splitter 318 as shown in Figures 3A and 3B. Alternatively, for example, the telecentric OCT scanning or probe beam 312b may pass in free-space directly to fold mirror 316 where it is reflected towards beam splitter 318.

The focussing lens assembly 314 acts as an optical interface for the telecentric OCT beam 312b to the microscope objective lens 210. The scanning mirror assembly could in some embodiments be used without a focussing lens assembly 314 but this would require the sample to be placed at the intermediate image plane 700 at which the telecentric OCT beam 312b is focussed on when it emerges from the scanning mirror assembly. So in order to use the OCT scanner 206 without the focusing lens assembly the sample would need to be somehow placed at the intermediate image plane 700. As shown in the OCT scanner example embodiment of Figures 3A, 3B and 4 for example, a lens is required in order to optically couple to the microscope objective lens 210 or alternatively, the objective lens 210 would need a much shorter focal length. Such a short focal length would not be conducive to surgical applications. However, in some embodiments of the disclosed technology, the OCT scanner may omit a focussing lens assembly 314 if it is used for another type of application. For example, an OCT scanner 206 used for eye imaging, especially animal eye imaging, may not require a focussing lens 314.

In some example embodiments of the OCT scanner adapter 206 used in a microscope for surgery, the focusing lens assembly 314 is fixed and set at the proper back focal length to collimate and expand an incident telecentric OCT beam 312b to have a 10.6mm collimated beam diameter at exit. As the OCT beam 312 is collimated on exiting the focussing assembly, it will focus at the focal plane of the microscope objective lens 210 as do the microscope optics.

Alternatively, by adjusting the location of the focusing lens assembly 314 relative to the intermediate image plane, the object distance is effectively being adjusted. This results in the focus location of the microscope objective lens 210 being changed accordingly for the OCT scan whereas the focus location remains fixed for the microscope optics.

A benefit of having a focussing lens assembly 314 in some embodiments of the OCT scanner, such as that of the example embodiments illustrated in Figures 3A, 3B and 4, is that if the surgeon moves an eyeball around during a surgical procedure, the OCT scanning system can keep the OCT focused at a designated anatomical feature using an appropriate autofocussing technique known in the art.

Another benefit of embodiments of the OCT scanner 206 which include a focussing lens assembly 314 is that it may be used in some situations even if the microscope optics are setup improperly by a user of the microscope 200, for example, by a surgeon or assistant. For example, if the microscope is non-parfocal, in other words if the oculars of the microscope are set to infinity for users with corrected eyesight via contacts or glasses, the microscope optics focus at the focal plane of the microscope objective lens. If, the microscope oculars are not set to accommodate the refractive error of a microscope user's eyesight, then some users may move the whole microscope, for example using handles 204a,b as shown in Figures 2A, 2B, to adjust or accommodate the refractive error in their eyesight. However, this movement of the microscope optics results in a scanned tissue sample or other scanned object of interest (for example, the eye under surgery) no longer being positioned at the actual focal plane of the microscope objective lens 210 provided by the OCT scanner system 206. In other words, if the microscope is improperly used then the focus of the OCT beam 312 may need to be adjusted accordingly to compensate using a focussing lens assembly such as focussing lens assembly 314.

In some embodiments, the OCT objective lens 510 shown in Figure 7 is a F2.7 Biconvex doublet lens. The objective lens 510 is coupled with a F19 Positive/Negative Meniscus doublet field lens 512 to turn the scanned collimated OCT beam 312 reflected from the surface 334 of the MEMS scanning mirror into an intermediate telecentric image plane shown in Figure 7. The OCT returned light beam passes via the OCT field lens, then the OCT objective lens, and is then reflected again via the reflective surface 334 of the MEMS mirror along the OCT output arm 518 via the OCT collimating lens 516 (see also Figure 5A) into an interferometer assembly (not shown in Figure 5A, see the SD-OCT system 100 of Figure 1).

In some embodiments, and as shown in the example embodiments of Figures 5A and 7, all air to glass interface surfaces such as surface 514 of the objective lens assembly 510 and the collimating lens 516 for the outward OCT beam 312 and returned OCT beam (not shown) are convex to eliminate back reflection artefacts in the OCT image.

In some embodiments, the total track length L within the scanning mirror assembly optical block is the sum of the length L1 from the end face 530 of the optical fibre 308a at the optical fibre ferrule 532 to the reflective surface 334 of the scanning mirror and L2, the track length on from the surface 334 to the telecentric image plane 700as shown in Figure 5A. The total track length L = L1 plus L2 is preferably less than 40mm.

In some embodiments, the optical path difference OPD at the sample being scanned has an OPD curvature greater than 100mm.

In some embodiments, the OCT scanning or probe beam is telecentric to better than 0.03 degrees incident angle.

In some embodiments, focusing system 314 provides a mechanism for the OCT beam 312 to be adjusted so that the OCT focal plane can be controlled within a ±30mm range to align with the microscope optical channel focal plane.

In some embodiments, the MEMS OCT scanner has a lateral X-Y profile where X is less than 42mm and Y is less than 35mm as Figure 5A shows schematically which allows the OCT scanner system housing to laterally fit within the lateral housing profile of the microscope optics carrier footprint. This is advantageous as it reduces obstructions in the sterile field for surgical applications. In some embodiments of the optical block implementing the scanning mirror assembly 310, the optical block dimensions are laterally a width, X, of around or equal to 40.6mm and a depth, Y, of around or equal to 34.5mm with a track length L of about 40mm or less.

In some embodiments, the scanning mirror assembly further comprises an optical angular displacement measurement system 156 for determining the angle of tilt of the reflective surface relative to incident light comprising at least: a point light source, a collimator lens assembly for collimating light from the point light source to form a collimated mirror position measuring light beam which is incident at the reflective surface; and a position sensitive detector, wherein the reflective surface is configured to reflect the incident collimated light beam in the first optical plane to form a reflected position measuring light beam which travels towards the position sensitive detector.

In some embodiments of the scanning mirror assembly 310 described hereinabove and with reference to Figures 5A and 5B of the drawings, a closed loop control of the position of the reflective surface of the MEMS mirror is provided by the position sensitive detector being configured to provide angular displacement measurement information to a controller configured to control an angle of tilt of the reflective mirror surface relative to an illuminating light beam. In some embodiments, the closed loop control uses a PID feedback loop to adjust the drive voltages to the MEMS based on position and also dampens the ringing artefacts caused by fast directional changes.

Advantageously, in some embodiments, where the scanning mirror assembly comprises a scanning mirror assembly 310 in OCT scanning apparatus 206, an input beam comprises an OCT probe beam 312 which is reflected through optical components along an OCT probe beam arm of the scanning mirror assembly towards a sample or similar object of interest 116. The scanning mirror assembly 310 is configured to output the OCT probe beam 312 as a telecentric OCT probe beam towards a focal plane 154 at the sample and the optical path length from the optical source 102 of the OCT probe beam to the sample focal image plane 154 is configured to be equivalent to that followed by a reference OCT beam from the same OCT optical source 112 along a reference arm 103 of a connected interferometer OCT system 100 for 2D scanning of the sample area 116.

In some embodiments, the MEMS scanning mirror assembly 310 disclosed herein is provided as an optical block 310 in an OCT scanner adapter 206 for a surgical microscope 200 which forms part of a connected OCT system 100. Such an OCT scanner adapter 206 preferably has at least a lateral footprint X within or equal to the footprint of the surgical microscope housing and preferably has a length or depth footprint also within the footprint of the microscope. The OCT system 100 outputs an interference signal comprising the OCT scan data 146 to the image processor 148 of the OCT system 100. The image processor 148 then processes the interference signal 146, for example, it may perform a signal transform such as a Fourier transform which allows an OCT image showing internal scanned structures within the scanned area to be displayed in an image on a display 152.I This image can be generated in real-time in some embodiments so as to guide to a surgeon and/or guide other parties on one or more suitable displays 152 in some embodiments.

In some embodiments of the OCT scanner adapter 206, the OCT scanner adapter 206 is configured to be fixed to the undercarriage of a housing of microscope optics of a surgical microscope, wherein the OCT scanner adapter 206 adds less than 40mm, preferably less than 36mm, to the stack height of the surgical microscope.

In some embodiments of the OCT scanner adapter 206, the OCT scanner adapter 206 is configured to be fixed to an undercarriage of the microscope optics housing and aligns an objective lens 114, 210 of the OCT scanner adapter 206 with an optical channel of the microscope optics when the lateral footprint of the housing 208 of the OCT scanner adapter 206 sits within the lateral footprint of the housing 202 of the surgical microscope 200.

In this manner, a surgical microscope, for example, a surgical microscope 200 such as that shown in Figures 2A and 2B comprising microscope optics, a housing 202 containing the microscope optics, and an OCT scanner adapter 206 can be provided according to the disclosed technology where the OCT scanner adapter 206 includes a scanning mirror assembly according to the disclosed technology, for example, such as that shown by way of example in Figures 5A and 3B. The OCT scanner adapter 206 may be configured to output image data which is later input into an image processor of an OCT system such as the OCT system shown in Figure 1.

Some embodiments of the above disclosed micro-electro-mechanical system, MEMS, two-dimensional scanning mirror assembly (310) accordingly comprise a scanning mirror assembly having an optical design comprising a moveable MEMS scanning mirror having a reflective surface 334, a point light source 308a for a light beam, a collimating lens assembly 516 configured to receive light from the light source and output a collimated light beam with an exit beam diameter above a threshold towards the reflective surface 334, and an objective lens assembly 510, 512 via which a collimated light beam reflected from the reflective surface exists the scanning mirror assembly. The reflective surface 334 is configured to reflect an incident collimated light beam to form a probe beam 312 which exits the mirror assembly as a telecentric beam (312) towards a telecentric image plane with a resolution better than a threshold for the telecentric beam resolution. The optics of the scanning mirror assembly are configured to provide a total track length, L, from the point light source to the telecentric image plane (700) less than 40mm and the scanning mirror assembly may be provided in an optical block which is less than or equal to 41mm wide, preferably 40.6 mm wide and less than or equal to 35mm, preferably 34.5 mm long, excluding the optical fibre connector 308 and MEMS support 500 (see X and Y for the block dimensions shown in Figure 5A).

The terminology used herein is for the purpose of describing particular aspects only and is not intended to be limiting of the disclosure. As used herein, the singular forms "a," "an," and "the" are intended to include the plural forms as well, unless the context clearly indicates otherwise.

As used herein the term "and/or" includes any and all combinations of one or more of the associated listed items and may be abbreviated as "/".

It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the inventive concepts being set forth in the following claims.

## Claims

1. A micro-electro-mechanical system, MEMS, two-dimensional scanning mirror assembly (310), the scanning mirror assembly comprising scanning mirror optics having an optical design comprising at least:
a moveable reflective surface (334) of a MEMS scanning mirror, the reflective surface being moveable in two-dimensions;
a collimating lens assembly (516) configured to receive a scanning light beam from a scanning light source (308a) and output a collimated scanning light beam towards the reflective surface (334), wherein the collimated scanning light beam has an exit beam diameter from the collimating lens assembly (516);
an objective lens assembly (510, 512) via which the collimated scanning light beam reflected from the reflective surface (334) exits the scanning mirror assembly (310),
wherein the configuration of the scanning mirror assembly optics includes the reflective surface (334) being configured to reflect an incident collimated scanning light beam towards the objective lens assembly (510, 512) to form a telecentric scanning beam (312) which exits the mirror assembly telecentric towards a telecentric image plane (700), wherein as the reflective surface (334) moves during a scan, the point where the telecentric scanning light beam focusses in the telecentric image plane changes; and wherein at least positions and dimensions of the scanning mirror optics within the scanning mirror assembly are configured to minimise a track length, L, from the light source (308a) to the telecentric image plane (700).

2. The MEMS scanning mirror assembly of claim 1, wherein the positions and dimensions of the scanning mirror optics within the mirror assembly define a track length L from the light source (308a) to the telecentric image plane (700) which is less than or equal to 40mm.

3. The MEMS scanning mirror assembly of claim 1, wherein the scanning light source (308a) comprises an optical fibre end-face (532) providing a point light source and wherein a numerical aperture of the point light source and a focal length of the collimating lens assembly (516) configure the scanning light beam exit beam diameter from the collimating lens to be at least 3.1 mm.

4. The MEMS scanning mirror assembly of any one of the previous claims, wherein the telecentric scanning light beam resolution at the telecentric image plane is better than 6 microns.

5. The MEMS scanning mirror assembly of any one of the previous claims, wherein the reflective surface (334) is configured to move within +/- 5 degrees about each of two orthogonal axes, x, y, which intersect at an optical centre of the reflective surface.

6. The MEMS scanning mirror assembly of any one of the previous claims, wherein the objective lens assembly optics comprise an objective lens (510) and a field lens (512) and wherein the total track length, L, is dependent on a combined focal length of the objective lens assembly optics.

7. The MEMS scanning mirror assembly as claimed in claim 6, wherein the objective lens (510) comprises a F2.7 Biconvex doublet lens and the field lens (512) comprises a F19 positive/negative meniscus doublet field lens.

8. The MEMS scanning mirror system of any one of the previous claims, wherein the optical path difference, OPD, of each scanning sequential beam which forms a telecentric scanning beam output by the scanning mirror assembly (310) has a radius of curvature which is greater than 100mm.

9. The MEMS scanning mirror system of any one of the previous claims, wherein each telecentric scanning beam is telecentric to better than an incident angle of 0.03 degrees from normal to the telecentric image plane.

10. The MEMS scanning mirror assembly of any one of the previous claims, wherein the reflective surface (334) comprises a large-aperture gold-coated silicon mirror bonded to an underlying mirror moving mechanical structure of the MEMS scanning mirror assembly (310).

11. The MEMS scanning mirror assembly of any one of the previous claims, wherein the MEMS scanning mirror assembly (310) is provided as an optical block in an optical coherence tomography, OCT, scanner (206), wherein the scanning light beam comprises an OCT probe beam (312), and wherein the light source (308a) comprises an optical fibre (308a) having an end-face (532) which acts as a point light source for the OCT probe beam (312).

12. The MEMS scanning mirror assembly (310) of either one of claims 10 or 11, wherein the OCT probe beam provides a depth scan of a sample and OCT light returned from the sample has a lateral optical resolution equal or higher than 6µm.

13. The MEMS scanning mirror assembly of any one of claims 11 to 12, wherein the OCT scanner (206) is provided as an OCT scanner adapter (206) for a surgical microscope (200).

14. A spectral-domain optical coherence tomography scanner system (100) comprising an optical interferometer arrangement, the scanner system (100) comprising:
a coupler (104) connected to an illuminating arm (101) including a light source (102), a scan depth reference arm (103), a scanning arm (105), and a detection arm (107),
wherein an illuminating light beam from the optical source 102 enters the coupler (104) along arm illuminating arm (101),
wherein the illuminating light beam is split by the coupler (104) into a scan depth reference light beam which follows the scan depth reference arm (103) towards a reflective surface (108) and a scanning light beam which follows scanning arm (105) towards a sample being scanned,
wherein the scanning arm (105) includes a scanning mirror assembly (310) according to any one of claims 1 to 14 configured to move the scanning light beam across a sample, such that light returned from the sample is returned along the scanning arm (105) towards the coupler (104),wherein returned light from the scanning arm and returned light from the scan depth reference arm (103) is directed by the coupler (104) towards an interference detector (136) located in detector arm (107) which is configured to detect interference between the returned light from the scanning arm and the returned light from the scan depth reference arm (103), and
wherein the interference detector (136) is configured to output data comprising an interference signal of the returned light for image processing to generate a tomograph of the scanned sample.

15. The SD-OCT scanner system (100) of claim 14, further comprising an image processor configured to process a received OCT interference signal and to output an OCT image for display.
